(19) Europäisches Patentamt
European Patent Office
Office européen des brevets

(11) EP 1 966 123 B1

(12) # EUROPEAN PATENT SPECIFICATION

(45) Date of publication and mention of the grant of the patent:
**19.11.2014 Bulletin 2014/47**

(21) Application number: **06834988.5**

(22) Date of filing: **13.12.2006**

(51) Int Cl.:
***H01L 51/50*** (2006.01) ***C07C 211/61*** (2006.01)
***C09K 11/06*** (2006.01)

(86) International application number:
**PCT/JP2006/325318**

(87) International publication number:
**WO 2007/072838 (28.06.2007 Gazette 2007/26)**

(54) **AMINE COMPOUND, ORGANIC LIGHT-EMITTING DEVICE, AND ORGANIC BLUE-LIGHT-EMITTING DEVICE**

AMINVERBINDUNG, ORGANISCHE LICHTEMITTIERENDE VORRICHTUNG UND ORGANISCHE BLAUEMITTIERENDE VORRICHTUNG

COMPOSE AMINE, DISPOSITIF ORGANIQUE EMETTEUR DE LUMIERE ET DISPOSITIF ORGANIQUE EMETTEUR DE LUMIERE BLEUE

(84) Designated Contracting States:
**AT BE BG CH CY CZ DE DK EE ES FI FR GB GR HU IE IS IT LI LT LU LV MC NL PL PT RO SE SI SK TR**

(30) Priority: **20.12.2005 JP 2005366557**
**20.11.2006 JP 2006312927**

(43) Date of publication of application:
**10.09.2008 Bulletin 2008/37**

(73) Proprietor: **CANON KABUSHIKI KAISHA**
**Ohta-ku**
**Tokyo 146-8501 (JP)**

(72) Inventors:
- **NEGISHI, Chika**
**Ohta-ku, Tokyo 1468501 (JP)**
- **YAMADA, Naoki**
**Ohta-ku, Tokyo 1468501 (JP)**
- **OKINAKA, Keiji**
**Ohta-ku, Tokyo 1468501 (JP)**
- **NAKASU, Minako**
**Ohta-ku, Tokyo 1468501 (JP)**

(74) Representative: **TBK**
**Bavariaring 4-6**
**80336 München (DE)**

(56) References cited:
**EP-A1- 1 577 364** **WO-A1-2004/020372**
**JP-A- 10 251 633** **JP-A- 2000 016 973**
**JP-A- 2001 039 933** **JP-A- 2001 064 241**
**JP-A- 2002 367 786** **JP-A- 2004 311 424**
**JP-A- 2004 315 366** **JP-A- 2005 268 228**
**JP-A- 2005 285 410** **US-A1- 2003 157 364**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### TECHNICAL FIELD

The present invention relates to an organic light-emitting device, and more specifically, to a device that radiates light by applying an electric field to a thin film composed of an organic compound.

### BACKGROUND ART

An organic light-emitting device is a device including a thin film which contains a fluorescent organic compound or a phosphorescent organic compound and which is interposed between an anode and a cathode, wherein a hole and an electron are injected from respective electrodes.

Further, an exciton of the fluorescent compound or the phosphorescent compound is generated in the device, and the device uses light radiated when the exciton returns to its ground state.

The recent progress of an organic light-emitting device is significant, and it is suggested that the device can be applied to a wide variety of applications because of the following reasons. The device shows a high luminance at a low applied voltage. In addition, the device has a variety of emission wavelengths. Furthermore, the device can be a thin, light-weight light-emitting device with high-speed responsiveness.

However, at present, an optical output with additionally high luminance, or additionally high conversion efficiency has been needed.

Japanese Patent No. 2851185 discloses, as examples of a material for an organic light-emitting device such as an amino compound and an organic light-emitting device using the material, an organic light-emitting device using a diamino condensed aromatic ring derivative. In addition, Japanese Patent No. 3508984 and Japanese Patent Application Laid-Open No. 2000-016973 disclose, as other examples, organic light-emitting devices using a diaminofluorene derivative. In addition, Japanese Patent Application Laid-Open No. H10-251633 and Japanese Patent Application Laid-Open No. 2001-39933 disclose, as other examples, organic light-emitting devices using a diamino compound. Further, Japanese Patent Application Laid-Open No. 2005-320277 discloses, as another example, an organic light-emitting device using a diamino compound as a quarter phenylene derivative in which a central biphenylene group is of an ortho-substituted type.

EP 1 577 364 A1 discloses an electroluminescence device, by which high brightness is provided in a manner of forming a hole injection or transport layer using a material that lowers an energy barrier. The device includes an organic emitting layer between a cathode and an anode and a hole injection and/or transport layer between the anode and the organic emitting layer, wherein at least one of the hole injection and transport layers contains a biphenyl group which is substituted in its meta-positions by four amino groups whose eight substituents are selected from an independently substituted or non-substituted aromatic group, a heterocyclic group, an aliphatic group and hydrogen.

WO 2004/020372 A1 discloses a fluorene compound wherein the benzene rings are substituted with a substituted amino group. For example, compound AA-58 of WO2004/020372 A1 is a quaterphenyl with two substituted amino groups in the para-positions, the substituents of the amino groups being fluorine and benzene ring systems. The fluorene compound is used to provide an organic luminescent device having an optical output exhibiting a high luminance with an extremely high efficiency, and having an extremely high durability.

### DISCLOSURE OF THE INVENTION

The present invention has been made in order to solve the above-mentioned problems of the prior art, and an object of the present invention is to realize extremely high luminous efficiency and to provide an organic light-emitting device having an optical output with a long lifetime. Another object of the present invention is to provide an organic light-emitting device that can be easily produced at a relatively low cost.

The inventors of the present invention have made extensive studies in order to solve the above-mentioned problems. As a result, they have accomplished the present invention.

(1) According to the present invention, there is provided an amine compound represented by the following general formula (1):

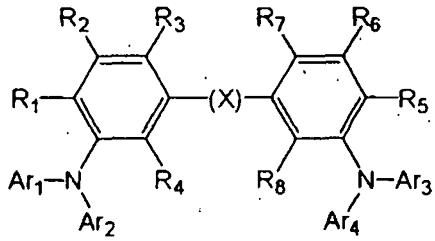

(1)

wherein X represents the following general formula (2):

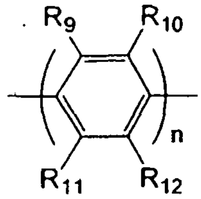

(2)

$Ar_1$, $Ar_2$, $Ar_3$ and $Ar_4$ each independently represent a substituted or unsubstituted aryl group, or a substituted or unsubstituted heterocyclic group, and may be the same or different, and at least one of $Ar_1$, $Ar_2$, $Ar_3$ and $Ar_4$ represents a substituted or unsubstituted fluorenyl group;
$R_1$ to $R_{12}$ each represent a hydrogen atom, a halogen atom, a substituted or unsubstituted alkyl group, wherein alkyl group includes a 1-adamantyl group, and a 2-adamantyl group, a substituted or unsubstituted alkoxy group, wherein alkox group includes a phenoxy group, a 4-tertiary butylphenoxy group, a benzyloxy group, and a thienyloxy group, a substituted or unsubstituted aryl group, or a substituted or unsubstituted heterocyclic group, and may be the same or different;

n represents an .integer of 0 to 10; and when a plurality of $R_9$'s, a plurality of $R_{10}$'s, a plurality of $R_{11}$'s, or a plurality of $R_{12}$' s, are present, the plurality of $R_9$'s, the plurality of $R_{10}$'s, the plurality of $R_{11}$'s, or the plurality of $R_{12}$'s may be the same or different, or two of $R_9$'s to $R_{12}$'s may form a ring, or X represents a single bond when n represents 0.
(2) According to the present.invention, there is also provided an amine compound according to the above item (1), wherein X in the general formula (1) is represented by the following general formula (3):

X= 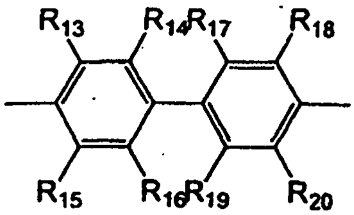

(3)

wherein $R_{13}$ to $R_{20}$ each represent a hydrogen atom, a halogen atom, a substituted or unsubstituted alkyl group, wherein alkyl groups includes a 1-adamantyl group and a 2-adamantyl group, a substituted or unsubstituted alkoxy group, wherein alkoxy group includes a phenoxy group, a 4-tertiary butylphenoxy group, a benzyloxy group, and a thienyloxy group, a substituted or unsubstituted aryl group, or a substituted or unsubstituted heterocyclic group, and may be the same different.
(3) According to the present invention, there is also provided an amine compound according to the above item (1), wherein X in the general formula (1) is represented by the following general formula (4):

X= 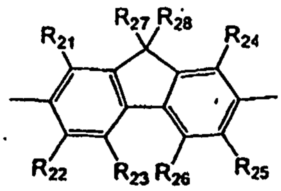

(4)

wherein $R_{21}$ to $R_{28}$ each represent a hydrogen atom, a halogen atom, a substituted or unsubstituted alkyl group, wherein alkyl groups includes a 1-adamantyl group and a 2-adamantyl group, a substituted or unsubstituted alkoxy group, wherein alkoxy group includes a phenoxy group, a 4-tertiary butylphenoxy group, a benzyloxy group, and a thienyloxy group, a substituted or unsubstituted aryl group, or a substituted or unsubstituted heterocyclic group, and may be the same or different.

(4) According to the present invention, there is also provided an amine compound according to the above item (1), wherein X in the general formula (1) is represented by the following general formula (5):

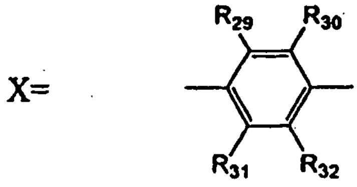

(5)

wherein $R_{29}$ to $R_{32}$ each represent a hydrogen atom, a halogen atom, a substituted or unsubstituted alkyl group, wherein alkyl group includes a 1-adamantyl group and a 2-adamantyl group a substituted or unsubstituted alkoxy group, wherein alkoxy group includes a phenoxy group, a 4-tertiary butylphenoxy group, a benzyloxy group and a thienyloxy group, a substituted or unsubstituted aryl group, or a substituted or unsubstituted heterocyclic group, and may be the same or different.

(5) According to the present invention, there is provided an amine compound according to Item (1), in which X in the general formula (1) represents a single bond.

(6) According to the present invention, there is provided an organic light-emitting device, including an organic compound layer interposed between a pair of electrodes composed of an anode and a cathode, at least one of the anode and the cathode being transparent or semi-transparent, wherein the organic compound layer contains an amine compound according to the above item (1).

(7) According to the present invention, there is provided an organic light-emitting device, including an organic compound layer as a hole transport layer or a hole injection layer interposed between a pair of electrodes composed of an anode and a cathode, at least one of the anode and the cathode being transparent or semi-transparent, wherein the hole transport layer or the hole injection layer contains an compound according to the above item (1).

(8) According to the present invention, there is provided an organic light-emitting device, including an organic compound layer as a light-emitting layer interposed between a pair of electrodes composed of an anode and a cathode at least one of the anode and the cathode being transparent or semi-transparent, wherein the light-emitting layer contains an amino compound according to Item (1).

The amine compound represented by the general formula (1) of the present invention emits light with a high efficiency at a low applied voltage. In addition, the device provides excellent durability.

BRIEF DESCRIPTION OF THE DRAWINGS

FIG. 1 is a sectional view showing an example of an organic light-emitting device of the present invention;
FIG. 2 is a sectional view showing another' example of the organic light-emitting device of the present invention;
FIG. 3 is a sectional view showing still another example of the organic light-emitting device of the present invention; and
FIG. 4 is a sectional view showing a further example of the organic light-emitting device of the present invention.
FIG. 5 is a sectional view showing a still further example of the organic light-emitting device of the present.invention.

BEST MODE FOR CARRYING OUT THE INVENTION

A compound represented by the above-described general formula (1) can be used mainly as a material for a hole transport layer or a material for a hole injection layer. The compound is characterized in that, when the compound is used as a material for a hole transport layer among such materials, the compound can be used to serve as an electron blocking layer, and the compound for use in a light-emitting device can provide the device having a high luminous efficiency and a long lifetime and have an effect particularly on highly efficient light emission.

A molecule of the compound represented by the general formula (1) was designed for the purpose of improving

the electron blocking property of a hole transport layer. In other words, the molecule was designed while the fact that the absolute value of the electron affinity of the hole transport layer should be sufficiently smaller than the absolute value of the electron affinity of a light-emitting layer was taken into consideration.

Details about the foregoing are as described below. Molecular design using a meta-phenylene group was performed for breaking a π-conjugated bond in a phenylene group on an amino substituent in a molecule of the compound. As a result, a material for a hole transport layer having a wide band gap ((the absolute value of the ionization potential of a hole transport layer) - (the absolute value of the electron affinity of the hole transport layer)) (hereinafter referred to as "Y") and a small absolute value of an electron affinity, and a hole transport layer using the material can be formed. In addition, in order that light emission with additionally high efficiency may be expected, the band gap can be adjusted by introducing the substituents $R_1$ to $R_{32}$ onto the phenylene group on the amino substituent. Further, the band gap, the ionization potential, and the electron affinity can be adjusted by transforming the substituents $Ar_1$ to $Ar_4$ on the respective amino groups.

Detailed, extensive investigation conducted by the inventors of the present invention has found that luminous efficiency tends to be larger as ((the absolute value of the electron affinity of a host material for use in a light-emitting layer) - (the absolute value of the electron affinity of a material for a hole transport layer)) (hereinafter referred to as "Z") becomes larger. It has also been found that, when Z is 0.42 eV or more and Y is 3.20 eV or more, an effect of improving the luminous efficiency is particularly significant. This is because the leak of an exciton from the light-emitting layer to the hole transport layer can be suppressed better as Y becomes larger. A compound described in the present invention can maintain Y of 3.20 eV or more because the compound uses a meta-phenylene group for cutting a n-conjugated bond.

In addition, it has been found that the increase of the value of Y is effective for an improvement in electron blocking property of the hole transport layer, that is, the increase of the value of Z. Further, it has been found that an improvement in electron blocking property of the hole transport layer requires the widening of a difference in band gap between the hole transport layer and a host of the light-emitting layer. In addition, detailed device evaluation has found that the achievement of highly efficient light emission and a long lifetime requires Y of 3.20 eV or more, Z of 0.42 eV or more, and a difference in band gap between the hole transport layer and the host of the light-emitting layer of 0.25 eV or more. Further, it has been found that a difference in band gap between the hole transport layer and the host of the light-emitting layer of 0.27 eV or more and Z of 0.48 eV or more are more preferable in order that those effects may be exerted. In addition, it has been found that such effects are particularly effective for a blue organic light-emitting device having a wide energy gap among an organic red-light-emitting device, an organic green-light-emitting device, and the organic blue-light-emitting device. As shown in examples and comparative examples, the above numerical values for Y, Z and the like were derived as a result of detailed investigation and discussion.

In addition, when a fluorescent material or a phosphorescent material is used in a light-emitting layer, each of a blue-light-emitting layer, a green-light-emitting layer, and a red-light-emitting layer can be transformed into a hole transport layer having an appropriate ionization potential and an appropriate electron affinity. In addition, when a fluorene derivative group is introduced into any one of $Ar_1$ to $Ar_4$, carrier transport property can be improved, Tg can be increased, and a material having good thermostability can be obtained.

In addition, when a phosphorescent material is used in a light-emitting layer, a hole transport layer in contact with the light-emitting layer desirably has an energy higher than that of the T1 level of the lowest excited triplet state of the phosphorescent material. To this end, a biphenylene group, fluorenyl group or phenylene group having a high T1 energy level, or a single bond is preferably introduced into X. More preferable examples include a phenylene group and a single bond. In addition, in consideration of the injection property of a hole from an anode, a biphenylene group or phenylene group represented by X is preferably para-substituted because the absolute value of the ionization potential of the hole transport layer must be small.

In addition, the compound represented by the general formula (1) can be used also as a material for a blue-light-emitting layer having high color purity. The present invention has been made by molecular design based on such discussion as described above.

Hereinafter, the present invention will be described in detail.

Specific examples of the substituents in the general formula (1) will be shown below.

According to the general formula (1), examples of the substituted or unsubstituted alkyl group include, of course not limited to, a methyl group, an ethyl group, a n-propyl group, an isopropyl group, an n-butyl group, a tert-butyl group, a sec-butyl group, an octyl group, a 1-adamantyl group, and a 2-adamantyl group.

Examples of the substituted or unsubstituted aryl group include, of course not limited to, a phenyl group, a naphthyl group, a pentalenyl group, an indenyl group, an azulenyl group, an anthryl group, a pyrenyl group, an indacenyl group, an acenaphthenyl group, a phenanthryl group, a phenalenyl group, a fluoranthenyl group, an acephenanthyl group, an aceanth.ryl group, a triphenylenyl group, a chrysenyl group, a naphthacenyl group, a perylenyl group, a pentacenyl group, a biphenyl group, a terphenyl group, and a fluorenyl group.

Examples of the substituted or unsubstituted heterocyclic group include, of course not limited to, a thienyl group, a pyrrolyl group, a pyridyl group, an oxazolyl group, an oxadiazolyl group, a thiazolyl group, a thiadiazolyl group, a

terthienyl group, a carbazolyl group, an acridinyl group, and a phenanthrolyl group.

Examples of the halogen atom include fluorine, chlorine, bromine, and iodine.

Examples of the substituted or unsubstituted alkoxy group include, of course not limited to, a substituted or unsubstituted methoxy group, ethoxy group, propoxy group, 2-ethyloctyloxy group, phenoxy group, 4-tertiary butylphenoxy group, benzyloxy group, and thienyloxy group.

Examples of substituents which the above-mentioned substituents may have include: alkyl groups such as a methyl group, an ethyl group, and a propyl group; aralkyl groups such as a benzyl group and a phenethyl group; aryl groups such as a phenyl group and a biphenyl group; heterocyclic groups such as a thienyl group, a pyrrolyl group, and a pyridyl group; amino groups such as an N,N-dimethylamino group, an N,N-diethylamino group, an N,N-dibenzylamino group, an N,N-diphenylamino group, an N,N-ditolylamino group, and an N,N-dianisolylamino group; alkoxyl groups such as a methoxyl group, an ethoxyl group, a propoxyl group, and a phenoxyl group; a cyano group; and halogen atoms such as fluorine, chlorine, bromine, and iodine.

Next, exemplified compounds are shown below as representative examples of the compound represented by the general formula (1); provided that the compound is not limited to these compounds.

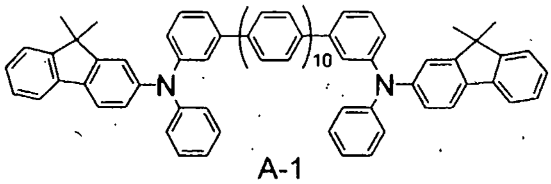

A-1

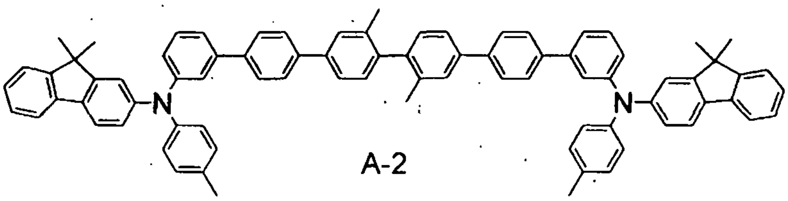

A-2

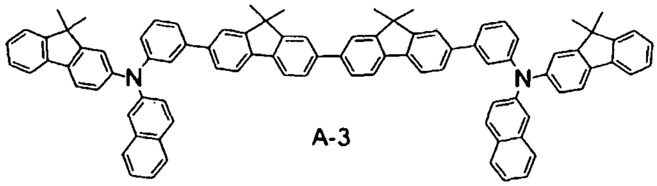

A-3

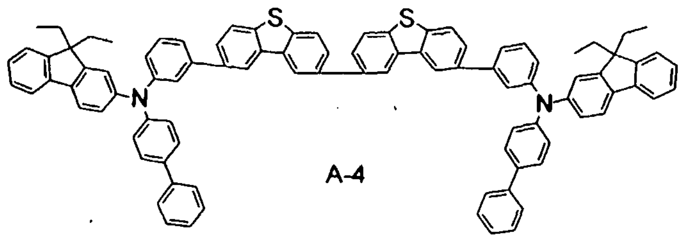

A-4

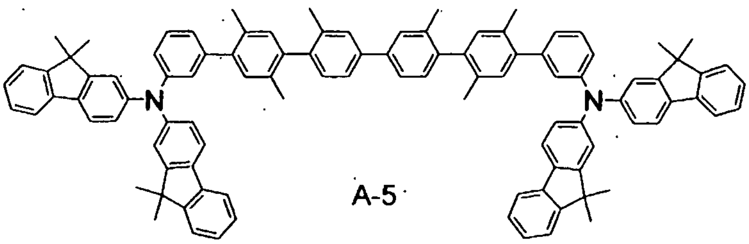

A-5

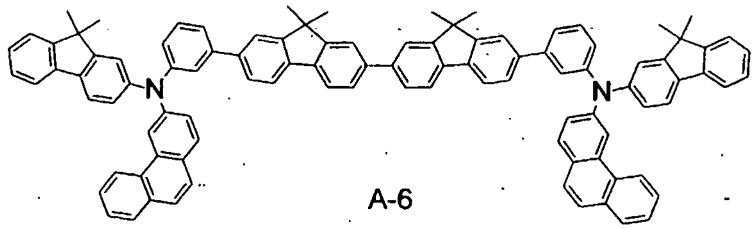

A-6

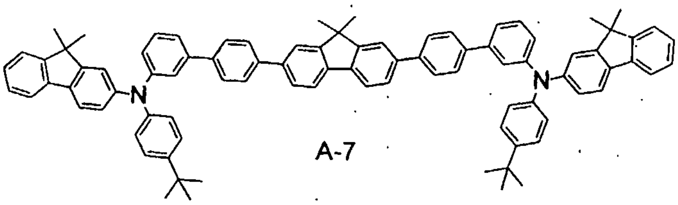

A-7

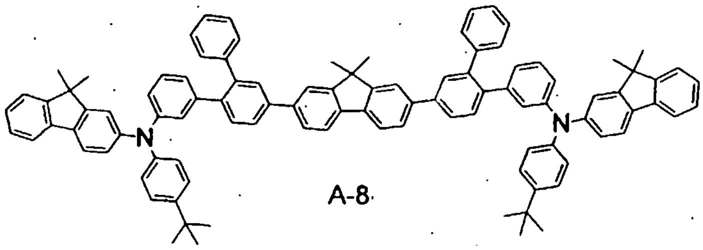

A-8

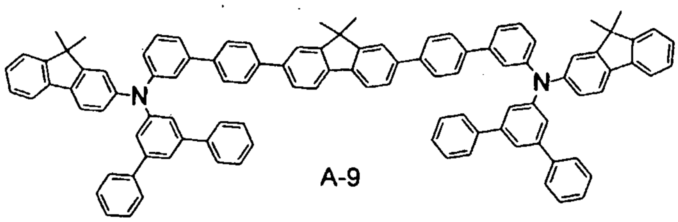

A-9

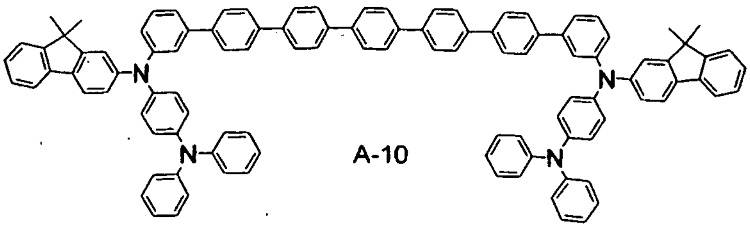

A-10

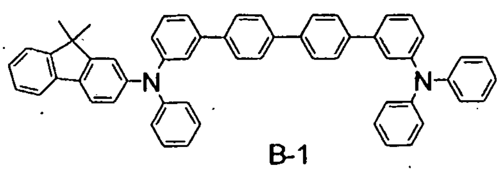

B-1

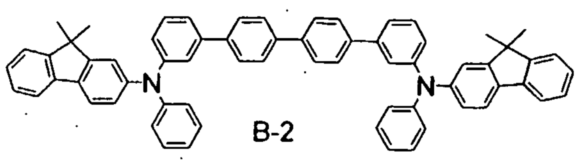

B-2

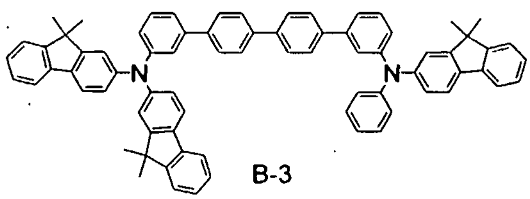

B-3

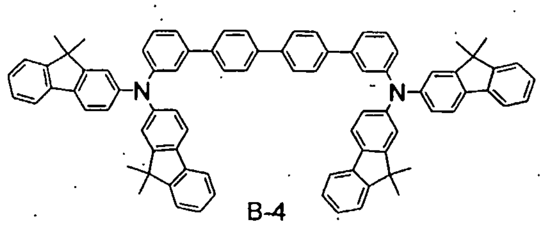

B-4

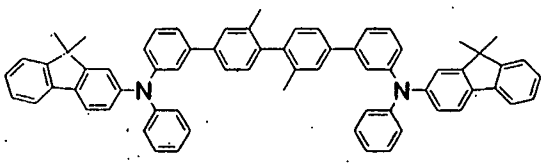

B-5

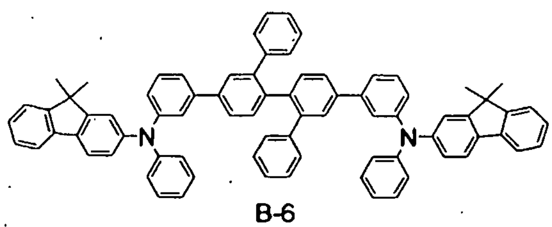

B-6

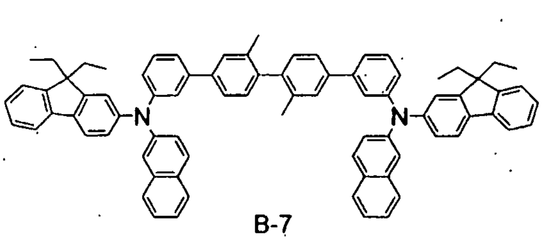

B-7

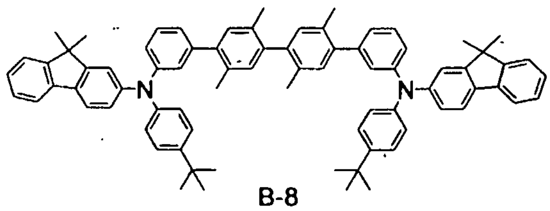

B-8

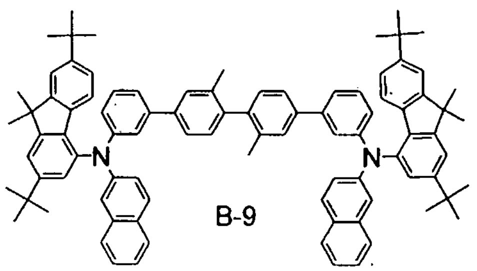

B-9

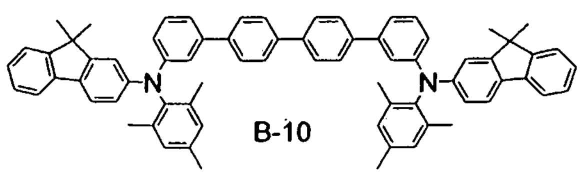

B-10

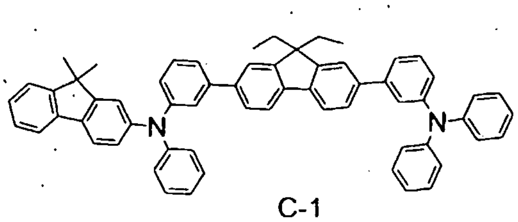

C-1

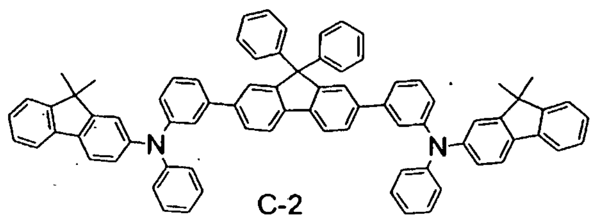

C-2

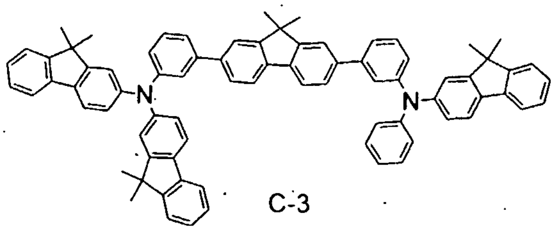

C-3

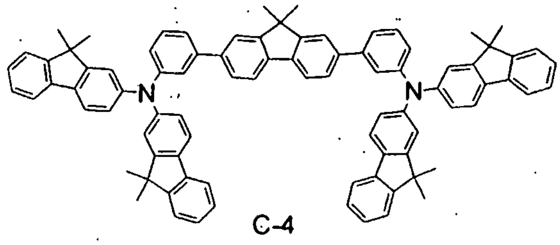

C-4

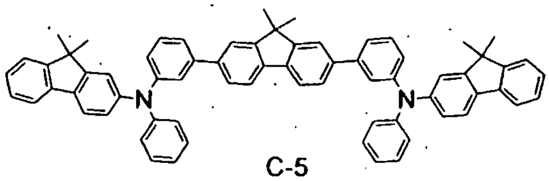
C-5

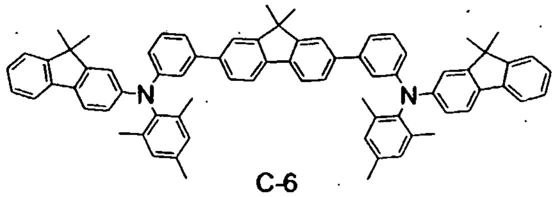
C-6

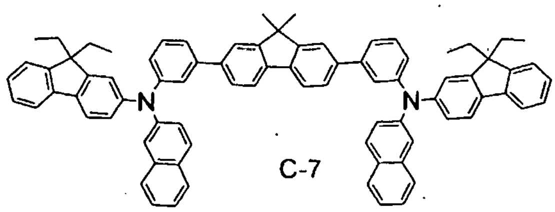
C-7

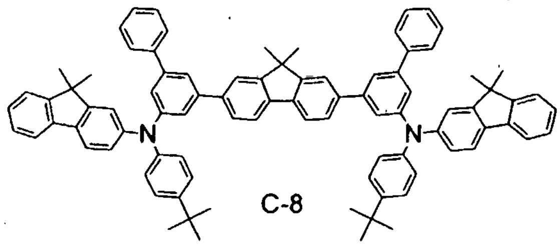
C-8

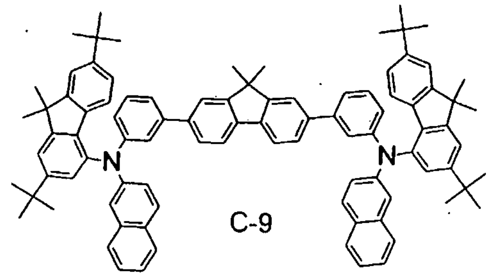
C-9

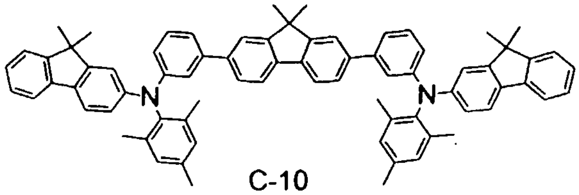
C-10

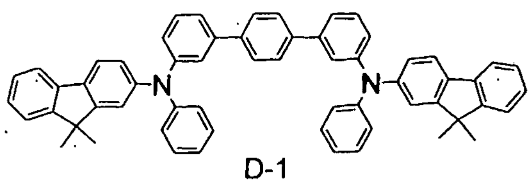
D-1

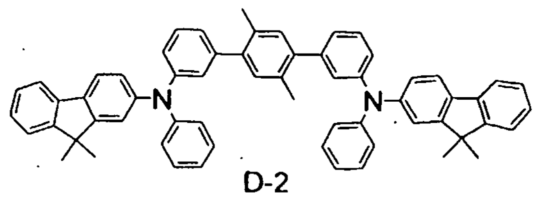
D-2

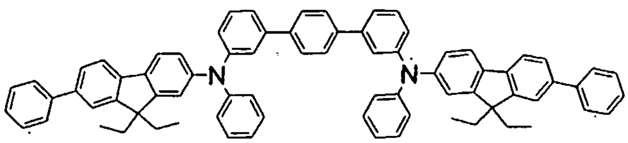
D-3

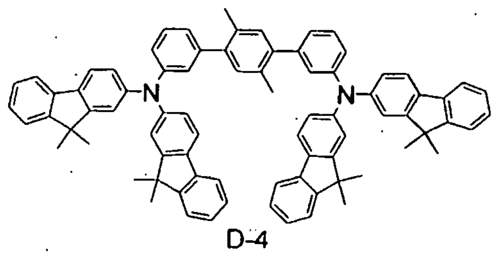
D-4

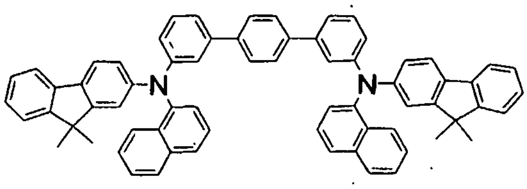

D-5

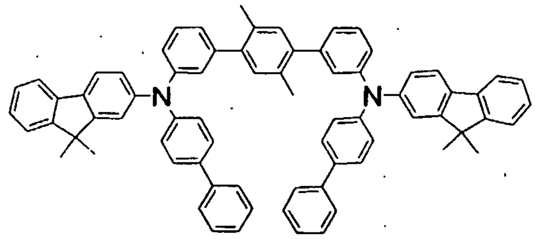

D-6

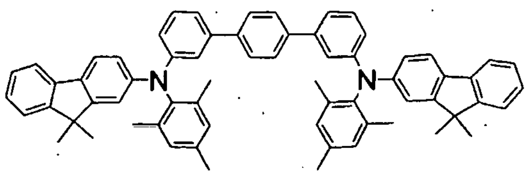

D-7

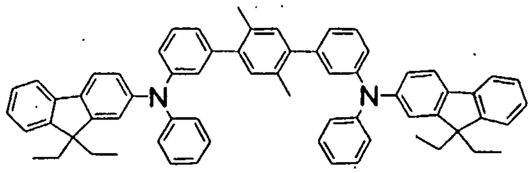

D-8

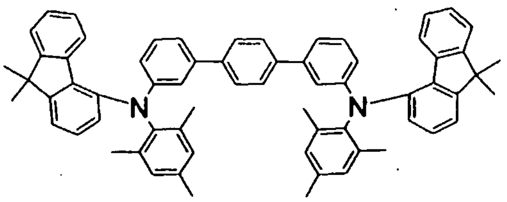

D-9

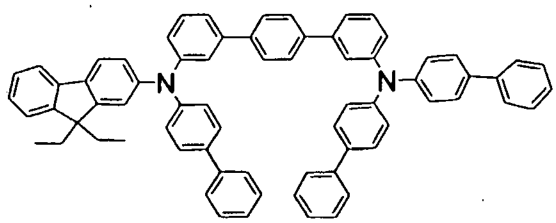

D-10

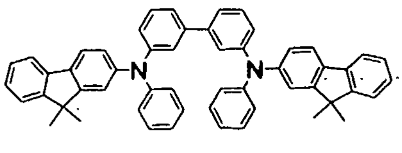

E-1

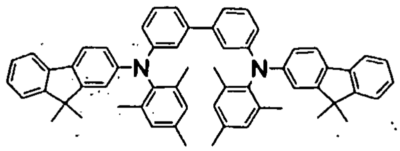

E-2

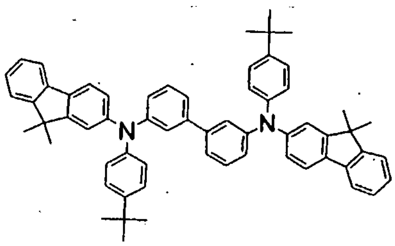

E-3

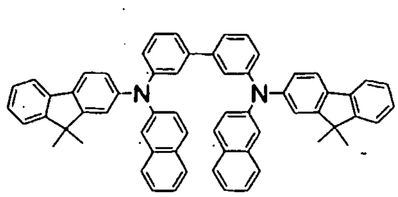

E-4

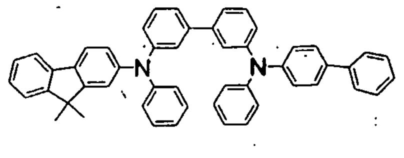

E-5

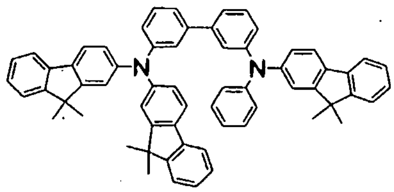

E-6

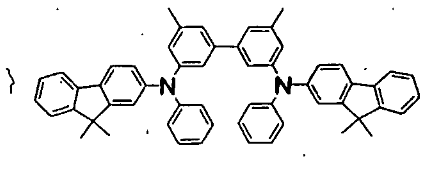

E-7

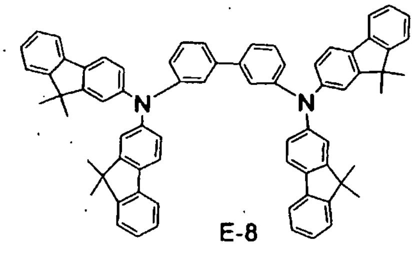

E-8

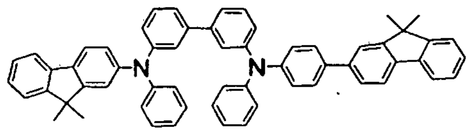

E-9

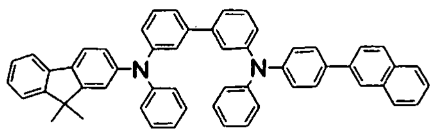

E-10

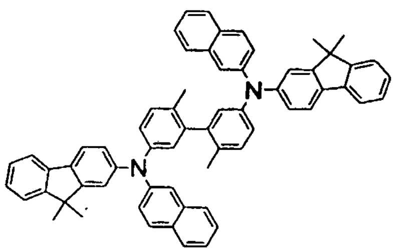

E-11

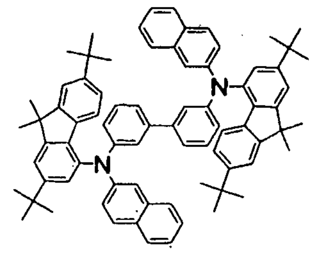

E-12

The compound represented by the general formula (1) of the present invention can be synthesized by a generally known method. The compound can be obtained by a synthesis method such as a Suzuki coupling method using a palladium catalyst (for example, Chem. Rev. 1995, 95, 2457-2483) or a Yamamoto method using a nickel catalyst (for example, Bull. Chem. Soc. Jpn. 51, 2091, 1978).

The compound represented by the general formula (1) of the present invention is a compound superior to any conventional compound in electron transport property, light emission property, and durability. In addition, the compound is useful in a layer containing an organic compound of an organic light-emitting device, in particular, each of a hole transport layer and a light-emitting layer. In addition, a layer formed by, for example, a vacuum deposition method or a solution application method hardly undergoes, for example, crystallization, and has excellent stability in time-dependent change.

Next, an organic light-emitting device of the present invention will be described in detail.

The organic light-emitting device of the present invention has at least a pair of electrodes composed of an anode and a cathode, and one or a plurality of layers each containing an organic compound, the one or plurality of layers being interposed between the pair of electrodes, wherein, at least one layer of the one or plurality of layers each containing an organic compound contains at least one kind of a compound represented by the general formula (1).

FIGS. 1 to 5 each show a preferable example of the organic light-emitting device of the present invention.

First, the reference numerals of the respective drawings will be described. Reference numeral 1 represents a substrate; 2, an anode; 3, a light-emitting layer; 4, a cathode; 5, a hole transport layer; 6, an electron transport layer; 7, a hole injection layer;' and 8, a hole/exciton blocking layer.

FIG. 1 is a sectional view showing an example of the organic light-emitting device of the present invention. FIG. 1 shows a constitution in which the anode 2, the light-emitting layer 3, and the cathode 4 are sequentially provided onto the substrate 1. The light-emitting device to be used here is useful in a case where the device itself has a hole-transporting property, an electron-transporting property, and light-emitting property alone or a case where compounds having respective properties are used as a mixture.

FIG. 2 is a sectional view showing another example of the organic light-emitting device of the present invention. FIG. 2 shows a constitution in which the anode 2, the hole transport layer 5, the electron transport layer 6, and the cathode 4 are sequentially provided onto the substrate 1. In this case, a material having one or both of hole-transporting property and electron-transporting property is used as a light-emitting substance in each layer. This case is useful when the device is used in combination with a mere hole-transporting substance or electron-transporting substance having no light-emitting properties. In addition, in this case, a light-emitting layer 3 is composed of the hole transport layed 5 or the electron transport layer 6.

FIG. 3 is a sectional view showing another example of the organic light-emitting device of the present invention. FIG. 3 shows a constitution in which the anode 2, the hole transport layer 5, the light-emitting layer 3, the electron transport layer 6, and the cathode 4 are sequentially provided onto the substrate 1. This constitution separates a carrier-transporting function and a light-emitting function. In addition, the device can be formed by timely using a compound having a hole-transporting property, a compound having an electron-transporting property, and a compound having a light-emitting property in combination, so that the degree of freedom in selection of materials extremely increases. In addition, various compounds different from each other in emission wavelength can be used. As a result, the range of luminescent colors can be widened. Furthermore, an emission efficiency can be improved by effectively trapping each carrier or exciton in the central light-emitting layer 3.

FIG. 4 is a sectional view showing another example in the organic light-emitting device of the present invention. FIG. 4 shows a constitution different from that shown in FIG. 3 in that the hole injection layer 7 is inserted on the side of the anode 2. The layer has an improved effect on adhesiveness between the anode 2 and the hole transport layer 5 or on hole injection property, and is effective for a reduction in voltage at which the device is driven.

FIG. 5 is a sectional view showing another example in the organic light-emitting device of the present invention. FIG. 5 shows a constitution different from that shown in FIG. 3 in that a layer (hole/exciton blocking layer 8) for inhibiting the escape of a hole or an exciton toward the side of the cathode 4 is inserted between the light-emitting layer 3 and the electron transport layer 6. The constitution is effective for an improvement in luminous efficiency when a compound having an extremely high ionization potential is used in the hole/exciton blocking layer 8.

The device constitutions shown in FIGS. 1 to 5 are merely very basic constitutions, and the constitution of an organic light-emitting device using the compound of the present invention is not limited to these constitutions. The device may adopt any one of various layer constitutions. For example, an insulating layer may be provided onto an interface between an electrode and an organic layer. Alternatively, an adhesive layer or an interference layer may be provided. In addition, a hole transport layer is constituted of two layers of different ionization potential.

The compound represented by the general formula (1) to be used in the present invention can be used in any one of the forms shown in FIGS. 1 to 5.

In the present invention, a compound represented by the general formula (1) is used particularly as a component of a light-emitting layer; a conventionally known, low-molecular-weight-based or polymer-based hole transport compound, luminescent compound, electron transport compound, or the like can be used together with the compound as required.

In an organic light-emitting device of the present invetion, a layer containing an amine compound represented by the general formula (1) and a layer composed of another organic compounds each generally form a thin film by a vacuum deposition method, an ionization deposition method, a sputtering method, or plasma. Alternatively, each layer was dissolved into an appropriate solvent to form a thin film by a known application method such as spin coating, a dipping method, a casting method, an LB method, an inkjet method, or the like. In particular, in the case where a thin film is formed by an application method, the film may also be formed in combination with an appropriate binder resin.

The binder resin may be selected from a wide variety of binder resin. Examples of the binder resin include, but not limited to, a polyvinyl carbazole resin, a polycarbonate resin, a polyester resin, a polyarylate resin, a polystyrene resin, an ABS resin, a polybutadine resin, a polyurethane resin, an acrylic resin, a methacrylic resin, a butyral resin, a polyvinyl acetal resin, a polyamide resin, a polyimide resin, a polyethylene resin, a polyethersulfone resin, a diallyl phthalate resin, a phenol resin, an epoxy resin, a silicone resin, a polysulfone resin, and a urea resin. Each of those resins may be used alone, or one or more of them may be mixed as a copolymer. Further, an additive such as a known plasticizer, antioxidant, or ultraviolet absorber may be used together if required.

An anode material preferably has as large a work function as possible. Examples of the anode material include a metal such as gold, platinum, silver, copper, nickel, palladium, cobalt, selenium, vanadium, or tungsten. In addition, each of alloys thereof and metal oxides such as tin oxide, zinc oxide, indium oxide, indium tin oxide (ITO), and indium zinc oxide may be used. Further, a conductive polymer such as polyaniline, polypyrrole, polythiophene, or polyphenylene sulfide may also be used. Each of those electrode substances may be used alone, or two or more of them may be used

in combination. Further, an anode may adopt a single layer construction or a multilayer construction.

On the other hand, a cathode material preferably has a small work function. Examples of the cathode material which can be used include: a metal such as lithium, sodium, potassium, calcium, magnesium, aluminum, indium, ruthenium, titanium, manganese, yttrium, silver, lead, tin, or chromium; and an alloy composed of a plurality of metals such as lithium-indium, sodium-potassium, magnesium-silver, aluminum-lithium, aluminum-magnesium, or magnesium-indium. A metal oxide such as indium tin oxide (ITO) may also be used. Each of those electrode substances may be used alone, or two or more of them may be used in combination. Further, a cathode may adopt a single layer construction or a multilayer construction.

Further, at least either of an anode or a cathode may desirably be transparent or semi-transparent.

A substrate to be used in the present invention is not particularly limited; provided that an opaque substrate such as a metallic substrate or a ceramic substrate, or a transparent substrate such as glass, quartz, or a plastic sheet is used. In addition, a luminescent color can be controlled by using a color filter film, a fluorescent color conversion filter film, a dielectric reflective film, or the like as the substrate.

In addition, a luminescent color can be controlled by using a color filter film, a fluorescent color conversion filter film, a dielectric reflective film, or the like as the substrate. Alternatively, a device can be produced by producing a thin film transistor (TFT) on a substrate and by connecting the TFT to the substrate.

In addition, with regard to the direction in which light is extracted from the device, both a bottom emission constitution (constitution in which light is extracted from a substrate side) and a top emission constitution (constitution in which light is extracted from the side opposite to the substrate) are available.

It should be noted that the produced device may be provided with a protective layer or a sealing layer for the purpose of preventing the device from contacting with, for example, oxygen or moisture. Examples of the protective layer include: an inorganic material film such as a diamond thin film, a metal oxide, or a metal nitride; a polymer film such as a fluorine resin, polyparaxylene, polyethylene, a silicone resin, or a polystyrene resin; and a photocurable resin. In addition, the device itself may be covered with, for example, glass, a gas impermeable film, or a metal, and packaged with an appropriate sealing resin.

An ionization potential as specified in the present invention is defined as energy needed for an electron at the highest occupied molecular orbital (HOMO) level of a compound to be released to a vacuum level. Meanwhile, an electron affinity is defined as energy with which an electron at a vacuum level drops to the lowest unoccupied molecular orbital (LUMO) level of a substance to stabilize.

An ionization potential can be directly measured by ultraviolet photoelectron spectroscopy (UPS) or by using a low-energy electron spectrometer (measuring instrument name AC-1, AC-2, or AC-3 manufactured by RIKENKEIKI CO., LTD). Alternatively, the ionization potential can be determined from, for example, the measurement of an oxidation potential by a cyclic voltammetry method.

In the present invention, a value for an ionization potential was defined as a value measured with an AC-1 manufactured by RIKENKEIKI CO., LTD.

An electron affinity is defined by the following expression:

$$\text{(Electron affinity)} = \text{(Ionization potential)} - \text{(Band gap)}.$$

The band gap can be measured by, for example, a method involving: depositing an organic compound from the vapor onto glass to provide a deposited film having a thickness of about 50 nm; measuring the absorption spectrum of the deposited film; and converting a wavelength Y (nm) of an absorption edge of the spectrum into X (eV).

X can be determined by a conversion expression "X = 1,240/Y".

The electron affinity can be determined also from the measurement of a reduction potential by a cyclic voltammetry method.

A method of determining an electron affinity adopted in the present invention involved calculating the electron affinity from a measured value of a band gap by light absorption and the above ionization potential. A spectrophotometer U-3010 (manufactured by Hitachi High-Technologies Corporation) was used for measuring an absorption spectrum.

Hereinafter, the present invention will be described more specifically by way of the following Examples. However, the present invention is not limited to these Examples.

(Example 1)

Synthesis of Exemplified Compound C-5

a) Synthesis of intermediate compound

Compound (1-1) can be produced by an Ullmann reaction by using N-(9,9-dimethyl-fluorene-2-yl)-N-phenylamine and 3-bromoiodobenzene as raw materials. b) Synthesis of Exemplified Compound C-5

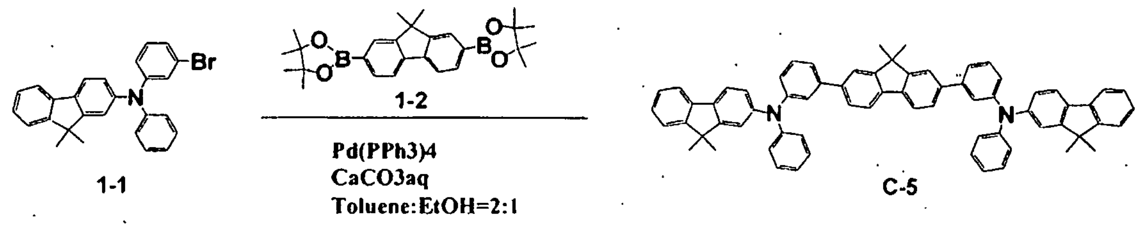


À 500-ml three-necked flask was prepared. 3.0 g (6.74 mmol) of Compound (1-2) were loaded into the flask. Further, 7.42 g (16.9 mmol) of Compound (1-1) were loaded into the flask. Further, 140 ml of toluene and 70 ml of ethanol were loaded into the flask, and an aqueous solution prepared by dissolving 3 g of calcium carbonate in 30 ml of water was dropped while the mixture was stirred in a nitrogen atmosphere at room temperature. Next, 0.39 g (0.34 mmol) of tetrakis(triphenylphosphine)palladium(0) was added. After the resultant had been stirred at room temperature for 30 minutes, the temperature of the resultant was increased to 70°C, and then the resultant was stirred for 6 hours. After the reaction, an organic layer was extracted with toluene, dried with anhydrous sodium sulfate, and purified with a silica gel column (mixed developing solvent of hexane and toluene), whereby 4.42 g of Exemplified Compound C-5 (white crystal) were obtained (72% yield). Matrix-assisted laser desorption/ionization time-of-flight mass spectrometry (MALDI-TOF MS) confirmed that the M+ of the compound was 912.4. Thermogravimetry (TG) and differential thermal analysis (DTA) were simultaneously performed to confirm that the compound had la melting point of 282°C. It was confirmed that the compound had an ionization potential of 5.46 eV, an electron affinity of 2.24 eV, and a band gap of 3.22 eV.

(Example 2)

Synthesis of Exemplified Compound B-2

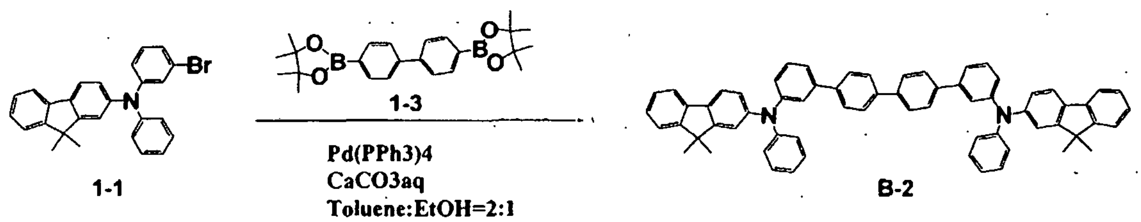


A 300-ml three-necked flask was prepared. 1.8 g (4.4 mmol) of Compound (1-3) were loaded into the flask. Further, 4.3 g (9.7 mmol) of Compound (1-1) were loaded into the flask. Further, 100 ml of toluene and 50 ml of ethanol were loaded into the flask, and an aqueous solution prepared by dissolving 2 g of calcium carbonate in 20 ml of water was dropped while the mixture was stirred in a nitrogen atmosphere at room temperature. Next, 0.25 g (0.22 mmol) of tetrakis(triphenylphosphine)palladium(0) was added. After the resultant had been stirred at room temperature for 30 minutes, the temperature of the resultant was increased to 70°C, and then the resultant was stirred for 9 hours. After the reaction, an organic layer was extracted with toluene, dried with anhydrous sodium sulfate, and purified with a silica gel column (mixed developing solvent of hexane and toluene), whereby 1.4 g of Exemplified Compound B-2 (white crystal) were obtained (74% yield). Matrix-assisted laser desorption/ionization time-of-flight mass spectrometry (MALDI-TOF MS) confirmed that the M+ of the compound was 872.4. Thermogravimetry (TG) and differential thermal analysis (DTA) were simultaneously performed to confirm that the compound had a melting point of 260°C. It was confirmed that the compound had an ionization potential of 5.46 eV, an electron affinity of 2.24 eV, and a band gap of 3.22 eV.

(Example 3)

Synthesis of Exemplified Compound D-2

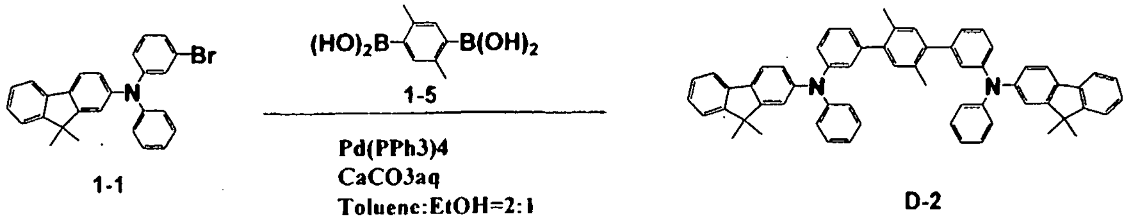


1-1 D-2

A 300-ml three-necked flask was prepared. 0.41 g (2.1 mmol) of Compound (1-5) were loaded into the flask. Further, 2.0 g (4.6 mmol) of Compound (1-1) were loaded into the flask. Further, 100 ml of toluene and 50 ml of ethanol were loaded into the flask, and an aqueous solution prepared by dissolving 2 g of calcium carbonate in 20 ml of water was dropped while the mixture was stirred in a nitrogen atmosphere at room temperature. Next, 0.13 g (0.11 mmol) of tetrakis(triphenylphosphine)palladium(0) was added. After the resultant had been stirred at room temperature for 30 minutes, the temperature of the resultant was increased to 70°C, and then the resultant was stirred for 8 hours. After the reaction, an organic layer was extracted with toluene, dried with anhydrous sodium sulfate, and purified with a silica gel column (mixed developing solvent of hexane and toluene), whereby 1.2 g of Exemplified Compound D-2 (white crystal) were obtained (68% yield). Matrix-assisted laser desorption/ionization time-of-flight mass spectrometry (MALDI-TOF MS) confirmed that the M+ of the compound was 824.4. Thermogravimetry (TG) and differential thermal analysis (DTA) were simultaneously performed to confirm that the compound had a melting point of 243°C. It was confirmed that the compound had an ionization potential of 5.50 eV, an electron affinity of 2.30 eV, and a band gap of 3.20 eV.

(Example 4)

Synthesis of Exemplified Compound E-5

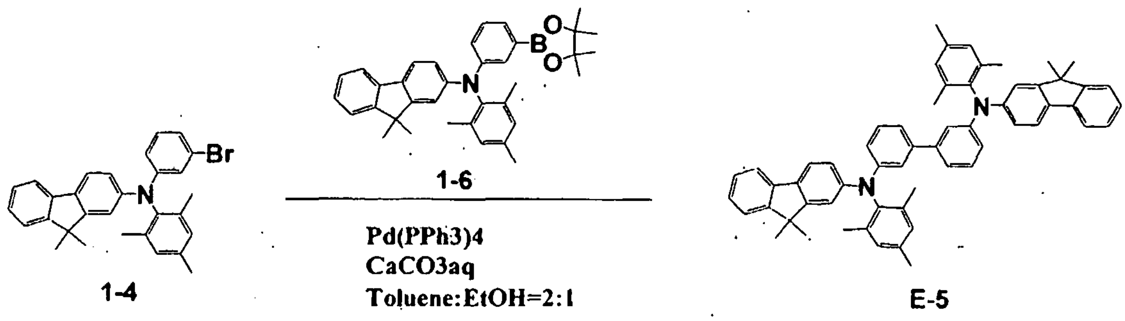


1-4 E-5

A 300-ml three-necked flask was prepared. 1.2 g (2.5 mmol) of Compound (1-6) were loaded into the flask. Further, 1.3 g (2.5 mmol) of Compound (1-4) were loaded into the flask. Further, 100 ml of toluene and 50 ml of ethanol were loaded into the flask, and an aqueous solution prepared by dissolving 2 g of calcium carbonate in 20 ml of water was dropped while the mixture was stirred in a nitrogen atmosphere at room temperature. Next, 0.29 g (0.25 mmol) of tetrakis(triphenylphosphine)palladium(0) was added. After the resultant had been stirred at room temperature for 30 minutes, the temperature of the resultant was increased to 80°C, and then the resultant was stirred for 10 hours. After the reaction, an organic layer was extracted with toluene, dried with anhydrous sodium sulfate, and purified with a silica gel column (mixed developing solvent of hexane and toluene), whereby 1.7 g of Exemplified Compound E-5 (white crystal) were obtained (82% yield). Matrix-assisted laser desorption/ionization time-of-flight mass spectrometry (MALDI-TOF MS) confirmed that the M+ of the compound was 804.4. Thermogravimetry (TG) and differential thermal analysis

(DTA) were simultaneously performed to confirm that the compound had a melting point of 228°C. It was confirmed that the compound had an ionization potential of 5.40 eV, an electron affinity of 2.16 eV, and a band gap of 3.24 eV.

(Example 5)

Synthesis of Exemplified Compound E-1

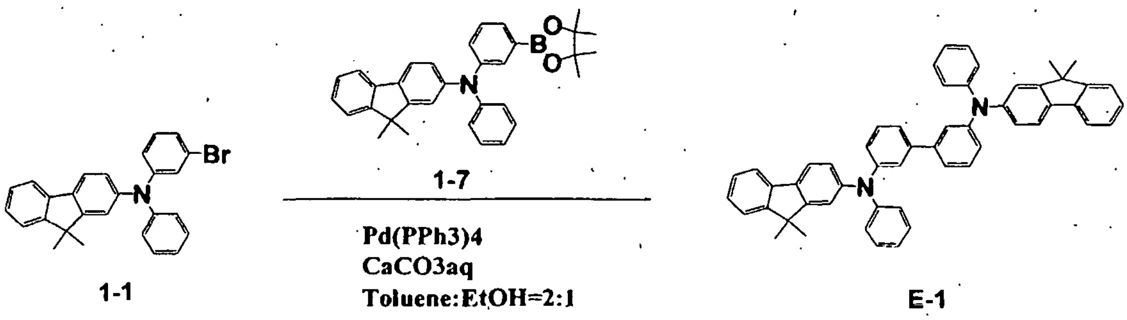


1-1 E-1

A 100-ml three-necked flask was prepared. 0.40 g (0.82 mmol) of Compound (1-7) were loaded into the flask. Further, 0.36 g (0.82 mmol) of Compound (1-1) were loaded into the flask. Further, 50 ml of toluene and 25 ml of ethanol were loaded into the flask, and an aqueous solution prepared by dissolving 1 g of calcium carbonate in 5 ml of water was dropped while the mixture was stirred in a nitrogen atmosphere at room temperature. Next, 0.12 g (0.1 mmol) of tetrakis(triphenylphosphine)palladium(0) was added. After the resultant had been stirred at room temperature for 30 minutes, the temperature of the resultant was increased to 70°C, and then the resultant was stirred for 5 hours. After the reaction, an organic layer was extracted with toluene, dried with anhydrous sodium sulfate, and purified with a silica gel column (mixed developing solvent of hexane and toluene), whereby 0.44 g of Exemplified Compound E-1 (white crystal) were obtained (75% yield). Matrix-assisted laser desorption/ionization time-of-flight mass spectrometry (MALDI-TOF MS) confirmed that the M+ of the compound was 720.3. Thermogravimetry (TG) and differential thermal analysis (DTA) were simultaneously performed to confirm that the compound had a melting point of 220°C. It was confirmed that the compound had an ionization potential of 5.46 eV, an electron affinity of 2.24 eV, and a band gap of 3.22 eV.

(Example 6)

An organic light-emitting device having a structure shown in FIG. 3 was produced by the following method.

Indium tin oxide (ITO) was formed by a sputtering method into a film having a thickness of 120 nm to serve as the anode 2 on a glass substrate as the substrate 1, and the resultant was used as a transparent, conductive supporting substrate. The substrate was subjected to ultrasonic cleaning with acetone and isopropyl alcohol (IPA) sequentially. Then, the substrate was subjected to boiling cleaning with IPA, followed by drying. Further, the substrate was subjected to UV/ozone cleaning. The resultant was used as a transparent, conductive supporting substrate.

Exemplified Compound C-5 was formed by a vacuum deposition method into a film having a thickness of 20 nm to serve as the hole transport material. The film was formed under conditions including: a degree of vacuum upon deposition of $1.0 \times 10^{-4}$ Pa; and a film formation rate of 0.1 nm/sec.

Next, Compound 2-1 shown below as a first compound and Compound 3-1 shown below as a second compound were co-deposited from the vapor (at a weight ratio of 90 : 10) onto the hole transport layer 5 to provide the light-emitting layer 3 having a thickness of 20 nm. The layer was formed under conditions including: a degree of vacuum upon deposition of $1.0 \times 10^{-4}$ Pa; and a film formation rate of 0.1 nm/sec.

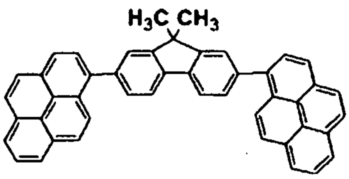

2-1

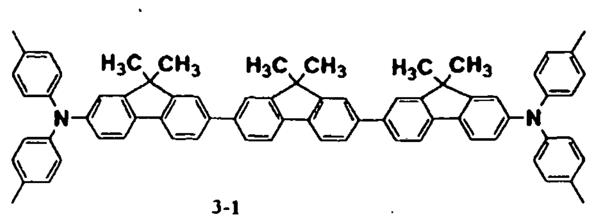

3-1

Compound 2-1 (ionization potential: 5.67 eV, electron affinity: 2.72 eV, band gap: 2.95 eV)

Compound 3-1 (ionization potential: 5.31 eV, electron affinity: 2.39 eV, band gap: 2.92 eV)

Further, bathophenanthroline (BPhen) was formed by a vacuum deposition method into a film having a thickness of 40 nm to serve as the electron transport layer 6. The film was formed under conditions including: a degree of vacuum upon deposition of $1.0 \times 10^{-4}$ Pa; and a film formation rate of 0.2 to 0.3 nm/sec.

Next, a metal layer film having a thickness of 0.5 nm was formed by a vacuum deposition method on the foregoing organic layer by using a deposition material composed of an aluminum-lithium alloy (lithium concentration: 1% by atom). Further, an aluminum film having a thickness of 150 nm was provided by a vacuum deposition method. Thus, an organic light-emitting device using the aluminum-lithium alloy film as an electron injection electrode (cathode 4) was produced. The films were each formed under conditions including: a degree of vacuum upon deposition of $1.0 \times 10^{-4}$ Pa; and a film formation rate of 1.0 to 1.2 nm/sec.

The resultant organic EL device was covered with a protective glass plate in a dry air atmosphere and sealed with an acrylic resin-based adhesive in order that the device might be prevented from deteriorating owing to the adsorption of moisture.

A voltage of 5.0 V was applied to the thus-obtained device by using the ITO electrode (anode 2) as a positive electrode and the Al electrode (cathode 4) as a negative electrode. As a result, the device was observed to emit blue light having a luminance of 2,800 cd/m$^2$ and an emission center wavelength of 458 nm.

Further, a voltage was applied for 100 hours while a current density was kept at 30 mA/cm$^2$ under a nitrogen atmosphere. As a result, an initial luminance of 1,720 cd/m$^2$ reduced to 1,280 cd/m$^2$ in 100 hours (the result is shown in Table 1 as a list).

(Examples 7 to 10)

Devices were each produced in the same manner as in Example 6 except that Exemplified Compound B-2, D-2, E-5, or E-1 was used instead of Exemplified Compound C-5, and were each evaluated in the same manner as in Example 6 (the results are shown in Table 1 as a list).

(Comparative Example 1)

A device was produced in the same manner as in Example 1 except that Comparative Compound 4-1 shown below was used instead of Exemplified Compound C-5, and was evaluated in the same manner as in Example 1. The device was observed to emit blue light having a luminance of 1,800 cd/m$^2$ and a wavelength of 458 nm at an applied voltage of 5.0 V. Further, a voltage was applied for 100 hours while a current density was kept at 30 mA/cm$^2$ under a nitrogen atmosphere. As a result, an initial luminance of 1,250 cd/m$^2$ reduced to 500 cd/m$^2$ in 100 hours (the result is shown in Table 1 as a list).

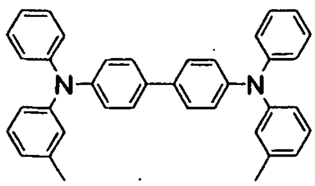

4-1

Compound 4-1 (ionization potential: 5.50 eV, electron affinity: 2.35 eV, band gap: 3.15 eV)

Table 1 below shows the list of the summary of the results of investigation on Examples 6 to 10 and Comparative Example 1.

Table 1

| | Compound No. | Initial | | Duration | | |
|---|---|---|---|---|---|---|
| | | Applied voltage (V) | Luminance (cd/m$^2$) | (Current density) 30.0 mA/cm$^2$ | Initial luminance (cd m$^2$) | Luminance after 100 hours (cd/m$^2$) |
| Example 6 | C-5 | 5 | 2800 | | 1720 | 1280 |
| 7 | B-2 | 5 | 2830 | | 1750 | 1350 |

(continued)

| | Compound No. | Initial | | Duration | | |
|---|---|---|---|---|---|---|
| | | Applied voltage (V) | Luminance ($cd/m^2$) | (Current density) $30.0\ mA/cm^2$ | Initial luminance ($cd\ m^2$) | Luminance after 100 hours ($cd/m^2$) |
| 8 | D-2 | 5 | 2260 | | 1590 | 980 |
| 9 | E-5 | 5 | 2900 | | 1800 | 1360 |
| 10 | E-1 | 5 | 2720 | | 1620 | 1160 |
| | | | | | | |
| Comparative Example 1 | 4-1 | 5 | 1800 | | 1250 | 500 |

In addition, values and evaluation represented by A to H of each of Exemplified Compounds C-5, B-2, D-2, E-5, and E-1, and Compound 4-1 used in Examples 6 to 10 and Comparative Example 1 are shown below.

(Table 2)

| | Compound No. | A | B | C | D | E | F | G | H |
|---|---|---|---|---|---|---|---|---|---|
| Example 6 | C-5 | 5.46 | 2.24 | 3.22 | 0.48 | 0.27 | 2800 | 74 | a |
| 7 | B-2 | 5.46 | 2.24 | 3.22 | 0.48 | 0.27 | 2830 | 77 | a |
| 8 | D-2 | 5.5 | 2.30 | 3.20 | 0.42 | 0.25 | 2260 | 61 | b |
| 9 | E-5 | 5.4 | 2.16 | 3.24 | 0.56 | 0.29 | 2900 | 75 | a |
| 10 | E-1 | 5.46 | 2.24 | 3.22 | 0.48 | 0.27 | 2720 | 72 | a |
| | | | | | | | | | |
| Comparative Example 1 | 4-1 | 5.50 | 2.35 | 3.15 | 0.37 | 0.20 | 1800 | 40 | c |

A: Absolute value for an ionization potential
B: Absolute value for an electron affinity
C: A - B (value for a band gap)
D: 2.72 (absolute value of the electron affinity of Compound 2-1 used as a host of a light-emitting layer) - B
E: C - 2.95 (value of the band gap of Compound 2-1 used as a host of a light-emitting layer)
F: Initial luminance at an applied voltage of 5 V (cd/m2)
G: Value of a luminance after 100 hours represented in a percentage unit when a current density is kept at $30\ mA/cm^2$ and an initial luminance is set to 1.
H: a ··· F is 2,700 or more, and G is 70 or more
b ··· F is 2,100 or more and less than 2,700, and G is 50 or more and less than 70
c ··· F is less than 2,100, and G is less than 50

Paying attention to the values for B and D described in Table 2 found that an emission luminance (F) tended to be larger as a value obtained by subtracting the absolute value for the electron affinity of a hole transport material (B) from the absolute value for the electron affinity of a host material for use in a light-emitting layer become larger. Further, it was found that, when D was 0.42 or more and B was smaller than 2.30, an increasing effect on the emission luminance was particularly significant (comparison between any one of Examples 6 to 10 and Comparative Example 1). In addition, it was also found that the emission luminance of Comparative Example 1 was smaller than that of any one of Examples 6 to 10. Those findings show that an improvement in electron blocking property in a hole transport layer can improve an emission luminance. That is, it was found that a large value for D contributed to an improvement in emission luminance. In addition, it was found from Table 2 that an increase in value for C was effective for an improvement in electron blocking property of the hole transport layer, that is, an increase in value for D. Further, it can be found from Table 2 that an improvement in electron blocking property of the hole transport layer requires the widening of a difference in band gap between the hole transport layer and a host for the light-emitting layer (E). The result of the evaluation of Comparative Example 1 for the above item H is c. In order that the result may be b or a, C must be 3.20 or more, D must be 0.42 or

more, and E must be 0.25 or more. Further, it was found that E of 0.27 or more and D of 0.48 or more were more preferable in order that those effects might be exerted. In addition, such effects are considered to be particularly effective for an organic blue-light-emitting device having a large light emission energy gap among an organic red-light-emitting device, an organic green-light-emitting device, and the organic blue-light-emitting device.

(Example 11)

An organic light-emitting device having a structure shown in FIG. 4 was produced by the following method.

Indium tin oxide (ITO) was formed by a sputtering method into a film having a thickness of 120 nm to serve as the anode 2 on a glass substrate as the substrate 1, and the resultant was used as a transparent, conductive supporting substrate. The substrate was subjected to ultrasonic cleaning with acetone and isopropyl alcohol (IPA) sequentially. Then, the substrate was subjected to boiling cleaning with IPA, followed by drying. Further, the substrate was subjected to UV/ozone cleaning. The resultant was used as a transparent, conductive supporting substrate.

A chloroform solution was prepared by using Compound 1 represented by the following constitutional formula 5-1 as a hole injection material in such a manner that the concentration of the compound would be 0.1 wt%.

The solution was dropped onto the above ITO electrode, and the whole was subjected to spin coating initially at a number of revolutions of 500 RPM for 10 seconds and then at a number of revolutions of 1,000 RPM for 1 minute, whereby a film was formed. After that, the resultant was dried for 10 minutes in a vacuum oven at 80°C, whereby the solvent in the thin film was completely removed. The first hole injection layer 7 thus formed had a thickness of 11 nm.

Next, Exemplified Compound C-5 as a hole transport layer was deposited from the vapor onto the hole injection layer 7 to provide an light-emitting layer 3 having a thickness of 20 nm. The layer was formed under conditions including: a degree of vacuum upon deposition of $1.0 \times 10^{-4}$ Pa; and a film formation rate of 0.1 nm/sec.

A light-emitting layer, an electron transport layer, and an A1 electrode were each produced in the same manner as in Example 6.

A voltage of 5.0 V was applied to the thus-obtained device by using the ITO electrode (anode 2) as a positive electrode and the A1 electrode (cathode 4) as a negative electrode. As a result, the device was observed to emit blue light having a luminance of 3,180 cd/m$^2$ and a center wavelength of 459 nm.

Further, a voltage was applied for 100 hours while a current density was kept at 30 mA/cm$^2$ under a nitrogen atmosphere. As a result, an initial luminance of 2,030 cd/m$^2$ reduced to 1,520 cd/m$^2$ in 100 hours.

(Examples 12 to 15).

The devices were each produced in the same manner as in.Example 11 by using Compound 5-1 in a hole injection layer except that Exemplified Compound B-2, D-2, E-5, or E-1 was used in a hole transport layer instead of Exemplified Compound C-5, and were each evaluated in the same manner as in Example 11 (the results are shown in Table 3 as a list).

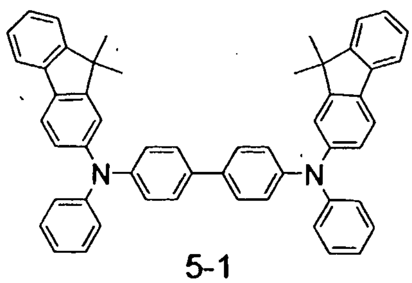

5-1

Table 3 below shows the list of the summary of the results of investigation on Examples 11 to 15.

(Table 3)

| | Hole injection layer material compound No. | Hole transport layer material compound No. | Initial | | Duration (Current density) 30.0 mA/cm$^2$ | Initial luminance (cd/m$^2$) | Luminance after 100 hours (cd/m$^2$) |
|---|---|---|---|---|---|---|---|
| | | | Applied voltage (V) | Luminance (cd/m$^2$) | | | |
| Example 11 | 5-1 | C-5 | 5 | 3180 | | 2030 | 1520 |

(continued)

| | Hole injection layer material compound No. | Hole transport layer material compound No. | Initial | | Duration (Current density) 30.0 mA/cm2 | Initial luminance (cd/m2) | Luminance after 100 hours (cd/m2) |
|---|---|---|---|---|---|---|---|
| | | | Applied voltage (V) | Luminance (cd/m2) | | | |
| 12 | 5-1 | B-2 | 5 | 320.0 | | 2110 | 1590 |
| 13 | 5-1 | D-2 | 5 | 2310 | | 1700 | 1200 |
| 14 | 5-1 | E-5 | 5 | 3140 | | 1980 | 1400 |
| 15 | 5-1 | E-1 | 5 | 3000 | | 1920 | 1360 |
| | | | | | | | |
| | | | | | | | |

As shown in Table 3, when Compound 5-1 was used as a material for a hole injection layer and a compound described in the present invention was used as a' material for a hole transport layer, a good value was obtained for each of the initial luminance of a device and the luminance after 100 hours of the device.

## Claims

1. An amine compound represented by the following general formula (1):

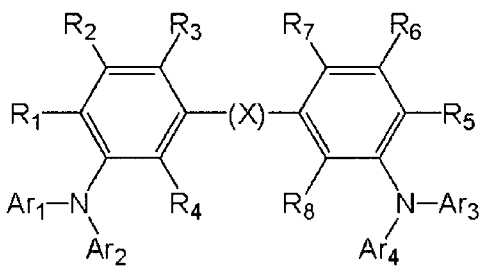

(1)

wherein X represents the following general formula (2):

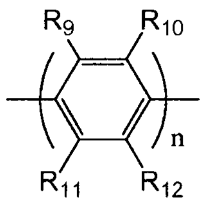

(2)

$Ar_1$, $Ar_2$, $Ar_3$ and $Ar_4$ each independently represent a substituted or unsubstituted aryl group, or a substituted or unsubstituted heterocyclic group, and may be the same or different, and at least one of $Ar_1$, $Ar_2$, $Ar_3$ and $Ar_4$ represents a substituted or unsubstituted fluorenyl group;
$R_1$ to $R_{12}$ each represent a hydrogen atom, a halogen atom, a substituted or unsubstituted alkyl group, wherein alkyl group includes a 1-adamantyl group and a 2-adamantyl group, a substituted or unsubstituted alkoxy group, wherein alkoxyl group includes a phenoxy group, a 4-tertiary butylphenoxy group, a benzyloxy group, and a thienyloxy group, a substituted or unsubstituted aryl group, or a substituted or unsubstituted heterocyclic group, and may be the same or different; and
n represents an integer of 0 to 10; and when a plurality of $R_9$'s, a plurality of $R_{10}$'s, a plurality of $R_{11}$'s, or a plurality of $R_{12}$'s are present, the plurality of $R_9$'s, the plurality of $R_{10}$'s, the plurality of $R_{11}$'s, or the plurality of $R_{12}$'s may be the same or different, or two of $R_9$'s to $R_{12}$'s may form a ring; or X represents a single bond when n represents 0.

2. An amine compound according to claim 1, wherein X in the general formula (1) is represented by the following general formula (3):

X= 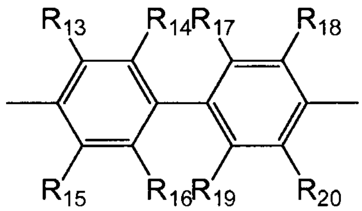 (3)

wherein $R_{13}$ to $R_{20}$ each represent a hydrogen atom, a halogen atom, a substituted or unsubstituted alkyl group, wherein alkyl group includes a 1-adamantyl group and a 2-adamantyl group, a substituted or unsubstituted alkoxy group, wherein alkoxyl group includes a phenoxy group, a 4-tertiary butylphenoxy group, a benzyloxy group, and a thienyloxy group, a substituted or unsubstituted aryl group, or a substituted or unsubstituted heterocyclic group, and may be the same or different.

3. An amine compound according to claim 1, wherein X in the general formula (1) is represented by the following general formula (4):

X= 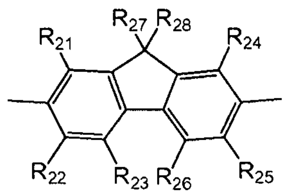 (4)

wherein $R_{21}$ to $R_{28}$ each represent a hydrogen atom, a halogen atom, a substituted or unsubstituted alkyl group, wherein alkyl group includes a 1-adamantyl group and a 2-adamantyl group, a substituted or unsubstituted alkoxy group, wherein alkoxyl group includes a phenoxy group, a 4-tertiary butylphenoxy group, a benzyloxy group, and a thienyloxy group, a substituted or unsubstituted aryl group, or a substituted or unsubstituted heterocyclic group, and may be the same or different.

4. An amine compound according to claim 1, wherein X in the general formula (1) is represented by the following general formula (5):

X= 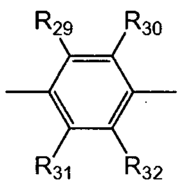 (5)

wherein $R_{29}$ to $R_{32}$ each represent a hydrogen atom, a halogen atom, a substituted or unsubstituted alkyl group, wherein alkyl group includes a 1-adamantyl group and a 2-adamantyl group, a substituted or unsubstituted alkoxy group, wherein alkoxy group includes a phenoxy group, a 4-tertiary butylphenoxy group, a benzyloxy group, and a thienyloxy group, a substituted or unsubstituted aryl group, or a substituted or unsubstituted heterocyclic group, and may be the same or different.

5. An amine compound according to claim 1, wherein X in the general formula (1) represents a single bond.

6. An amine compound according to claim 1, wherein the general formula (1) represents one selected from the group consisting of the following chemical formulas:

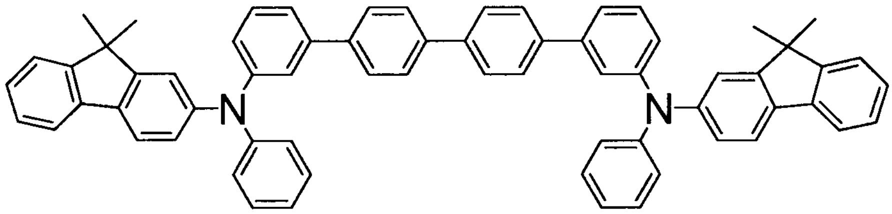

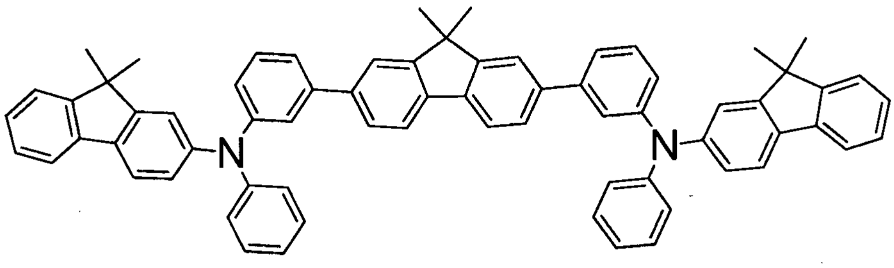

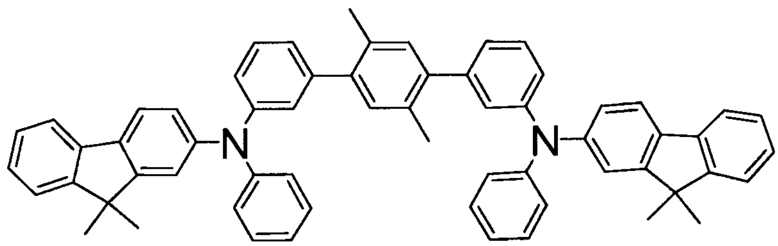

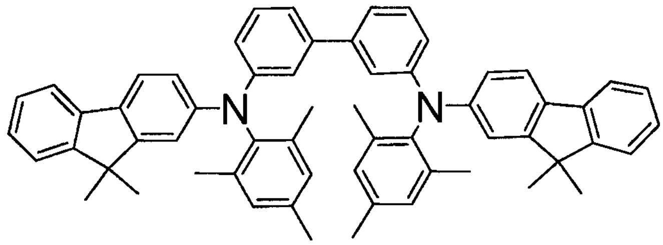

and

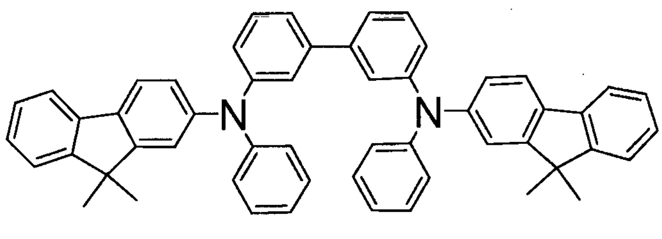

7. An organic light-emitting device, comprising an organic compound layer interposed between a pair of electrodes composed of an anode and a cathode, at least one of the anode and the cathode being transparent or semi-transparent, wherein the organic compound layer contains an amine compound according to claim 1.

8. An organic light-emitting device, comprising an organic compound layer as a hole transport layer or a hole injection layer interposed between a pair of electrodes composed of an anode and a cathode, at least one of the anode and the cathode being transparent or semi-transparent, wherein the hole transport layer or the hole injection layer contains an amine compound according to claim 1.

9. An organic light-emitting device, comprising an organic compound layer as a light-emitting layer interposed between a pair of electrodes composed of an anode and a cathode, at least one of the anode and the cathode being transparent or semi-transparent, wherein the light-emitting layer contains an amine compound according to claim 1.

10. A device comprising an opaque substrate and the organic light-emitting device according to claim 7.

11. The device according to claim 10, further comprising a color filter film.

## Patentansprüche

1. Aminverbindung, die durch die folgende allgemeine Formel (1) dargestellt ist:

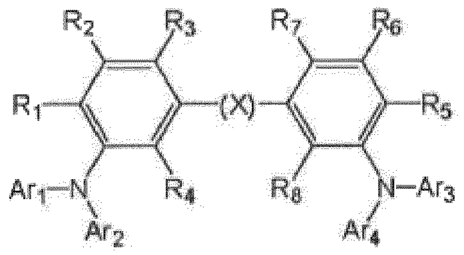

(1)

wobei X die folgende allgemeine Formel (2) darstellt:

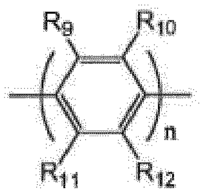

(2)

$Ar_1$, $Ar_2$, $Ar_3$ und $Ar_4$ stellen jeweils unabhängig eine substituierte oder unsubstituierte Arylgruppe oder eine substituierte oder unsubstituierte heterozyklische Gruppe dar, und können die gleichen oder unterschiedliche sein, und zumindest eines aus $Ar_1$, $Ar_2$, $Ar_3$ und $Ar_4$ stellt eine substituierte oder unsubstituierte Fluorenylgruppe dar;
$R_1$ bis $R_{12}$ stellen jeweils ein Wasserstoffatom, ein Halogenatom, eine substituierte oder unsubstituierte Alkylgruppe, wobei die Alkylgruppe eine 1-Adamantylgruppe und eine 2-Adamantylgruppe beinhaltet, eine substituierte oder unsubstituierte Alkoxygruppe, wobei die Alkoxylgruppe eine Phenoxygruppe, eine 4-tertiäre Butylphenoxygruppe, eine Benzyloxygruppe und eine Thienyloxygruppe beinhaltet; eine substituierte oder unsubstituierte Arylgruppe oder eine substituierte oder unsubstituierte heterozyklische Gruppe dar, und können die gleichen oder unterschiedliche sein;
n stellt eine ganze Zahl von 0 bis 10 dar; und wenn eine Mehrzahl an $R_9$, eine Mehrzahl an $R_{10}$, eine Mehrzahl an $R_{11}$ oder eine Mehrzahl an $R_{12}$ vorhanden sind, die Mehrzahl an $R_9$, die Mehrzahl an $R_{10}$, die Mehrzahl an $R_{11}$ oder die Mehrzahl an $R_{12}$ die gleichen oder unterschiedliche sein können, oder zwei von $R_9$ bis $R_{12}$ können einen Ring bilden; oder X stellt eine Einfachbindung dar, wenn n 0 darstellt.

2. Aminverbindung nach Anspruch 1, wobei X in der allgemeinen Formel (1) durch die folgende allgemeine Formel (3) dargestellt ist:

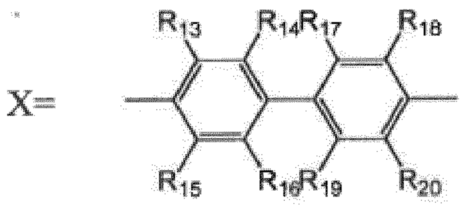

(3)

wobei $R_{13}$ bis $R_{20}$ jeweils ein Wasserstoffatom, ein Halogenatom, eine substituierte oder unsubstituierte Alkylgruppe, wobei Alkylgruppe eine 1-Adamantylgruppe und 2-Adamantylgruppe beinhaltet, eine substituierte oder unsubstituierte Alkoxygruppe, wobei Alkoxylgruppe eine Phenoxygruppe, eine 4-tertiäre Butylphenoxygruppe, eine Benzyloxygruppe und eine Thienyloxygruppe beinhaltet, eine substituierte oder unsubstituierte Arylgruppe, oder eine substituierte oder unsubstituierte heterozyklische Gruppe darstellen, und die gleichen oder unterschiedliche sein können.

**3.** Aminverbindung nach Anspruch 1, wobei X in der allgemeinen Formel (1) durch die folgende allgemeine Formel (4) dargestellt ist:

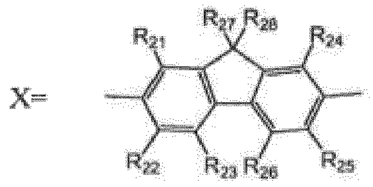

(4)

wobei $R_{21}$ bis $R_{28}$ jeweils ein Wasserstoffatom, ein Halogenatom, eine substituierte oder unsubstituierte Alkylgruppe, wobei Alkylgruppe eine 1-Adamantylgruppe und 2-Adamantylgruppe beinhaltet, eine substituierte oder unsubstituierte Alkoxygruppe, wobei Alkoxylgruppe eine Phenoxygruppe, eine 4-tertiäre Butylphenoxygruppe, eine Benzyloxygruppe und eine Thienyloxygruppe beinhaltet, eine substituierte oder unsubstituierte Arylgruppe, oder eine substituierte oder unsubstituierte heterozyklische Gruppe darstellen, und die gleichen oder unterschiedliche sein können.

**4.** Aminverbindung nach Anspruch 1, wobei X in der allgemeinen Formel (1) durch die folgende allgemeine Formel (5) dargestellt ist:

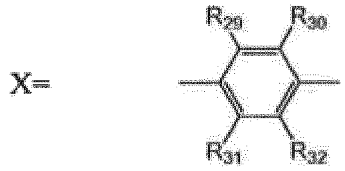

(5)

wobei $R_{29}$ bis $R_{32}$ jeweils ein Wasserstoffatom, ein Halogenatom, eine substituierte oder unsubstituierte Alkylgruppe, wobei Alkylgruppe eine 1-Adamantylgruppe und 2-Adamantylgruppe beinhaltet, eine substituierte oder unsubstituierte Alkoxygruppe, wobei Alkoxylgruppe eine Phenoxygruppe, eine 4-tertiäre Butylphenoxygruppe, eine Benzyloxygruppe und eine Thienyloxygruppe beinhaltet, eine substituierte oder unsubstituierte Arylgruppe, oder eine substituierte oder unsubstituierte heterozyklische Gruppe darstellen, und die gleichen oder unterschiedliche sein können.

**5.** Aminverbindung nach Anspruch 1, wobei X in der allgemeinen Formel (1) eine Einfachbindung darstellt.

**6.** Aminverbindung nach Anspruch 1, wobei die allgemeine Formel (1) eine darstellt, die aus der Gruppe ausgewählt ist, die aus den folgenden chemischen Formeln besteht:

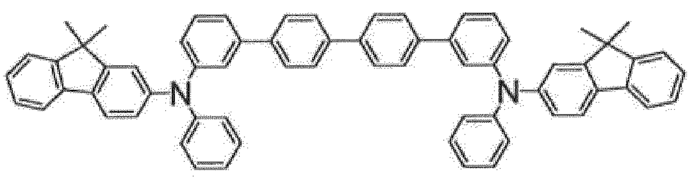

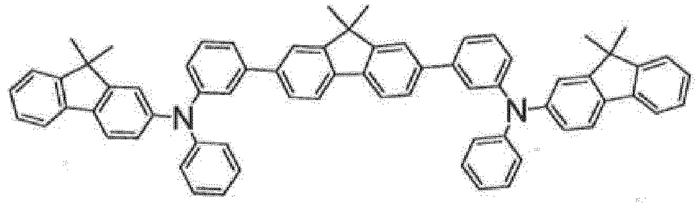

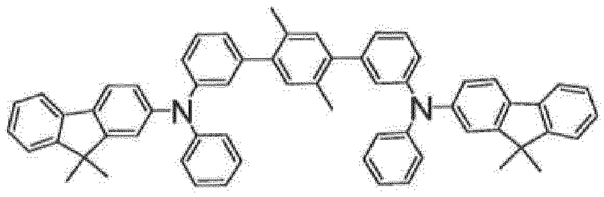

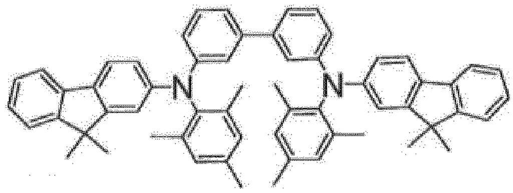

und

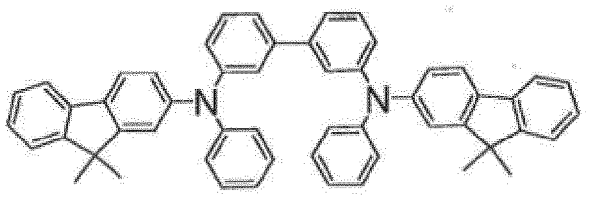

7. Organische lichtemittierende Vorrichtung, die eine organische Verbindungsschicht umfasst, die zwischen einem Paar von Elektroden zwischen angeordnet ist, die aus einer Anode und einer Kathode aufgebaut sind, wobei zumindest eine der Anode und der Kathode transparent oder halbtransparent ist, wobei die organische Verbindungsschicht eine Aminverbindung nach Anspruch 1 enthält.

8. Organische lichtemittierende Vorrichtung, die eine organische Verbindungsschicht als eine Lochtransportschicht oder als eine Lochinjektionsschicht umfasst, die zwischen einem Paar von Elektroden zwischen angeordnet ist, die aus einer Anode und einer Kathode aufgebaut sind, wobei zumindest eine der Anode und der Kathode transparent oder halbtransparent ist, wobei die Lochtransportschicht oder die Lochinjektionsschicht eine Aminverbindung nach Anspruch 1 enthält.

9. Organische lichtemittierende Vorrichtung, die eine organische Verbindungsschicht als eine lichtemittierende Schicht umfasst, die zwischen einem Paar von Elektroden zwischen angeordnet ist, die aus einer Anode und einer Kathode aufgebaut sind, wobei zumindest eine der Anode und der Katode transparent oder halbtransparent ist, wobei die lichtemittierende Schicht eine Aminverbindung nach Anspruch 1 enthält.

10. Vorrichtung, die ein opakes Substrat und die organische lichtemittierende Vorrichtung nach Anspruch 7 umfasst.

11. Vorrichtung nach Anspruch 10, die ferner einen Farbfilterfilm umfasst.

**Revendications**

1. Amine représentée par la formule générale (1) suivante :

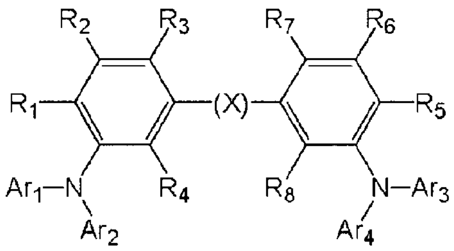

(1)

dans laquelle X répond à la formule générale (2) suivante :

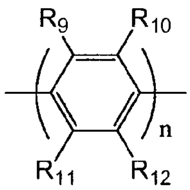

(2)

$Ar_1$, $Ar_2$, $Ar_3$ et $Ar_4$ représentent chacun indépendamment un groupe aryle substitué ou non substitué, ou un groupe hétérocyclique substitué ou non substitué, et peuvent être identiques ou différents, au moins un de $Ar_1$, $Ar_2$, $Ar_3$ et $Ar_4$ représentant un groupe fluorényle substitué ou non substitué ;

$R_1$ à $R_{12}$ représentent chacun un atome d'hydrogène, un atome d'halogène, un groupe alkyle substitué ou non substitué, ledit groupe alkyle comprenant un groupe 1-adamantyle et un groupe 2-adamantyle, un groupe alkoxy substitué ou non substitué, ledit groupe alkoxyle comprenant un groupe phénoxy, un groupe 4-tertiobutylphénoxy, un groupe benzyloxy et un groupe thiényloxy, un groupe aryle substitué ou non substitué ou un groupe hétérocyclique substitué ou non substitué, et peuvent être identiques ou différents ; et

n représente un nombre entier de 0 à 10 ; et, lorsqu'une pluralité de groupes $R_9$, une pluralité de groupes $R_{10}$, une pluralité de groupes $R_{11}$ ou une pluralité de groupes $R_{12}$ est présente, les groupes $R_9$, les groupes $R_{10}$, les groupes $R_{11}$ ou les groupes $R_{12}$ peuvent être identiques ou différents, ou bien deux des groupes $R_9$ à $R_{12}$ peuvent former un noyau ; ou bien X représente une liaison simple lorsque n est égal à 0.

**2.** Amine suivant la revendication 1, dans laquelle X dans la formule générale (1) répond à la formule générale (3) suivante :

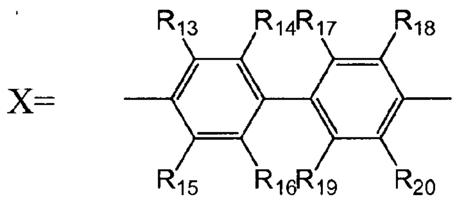

(3)

dans laquelle $R_{13}$ à $R_{20}$ représentent chacun un atome d'hydrogène, un atome d'halogène, un groupe alkyle substitué ou non substitué, ledit groupe alkyle comprenant un groupe 1-adamantyle et un groupe 2-adamantyle, un groupe alkoxy substitué ou non substitué, ledit groupe alkoxyle comprenant un groupe phénoxy, un groupe 4-tertiobutylphénoxy, un groupe benzyloxy et un groupe thiényloxy, un groupe aryle substitué ou non substitué ou un groupe hétérocyclique substitué ou non substitué, et peuvent être identiques ou différents.

**3.** Amine suivant la revendication 1, dans laquelle X dans la formule générale (1) répond à la formule générale (4) suivante :

X= 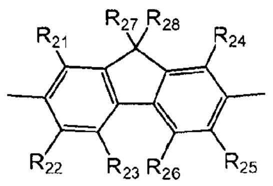 (4)

dans laquelle $R_{21}$ à $R_{28}$ représentent chacun un atome d'hydrogène, un atome d'halogène, un groupe alkyle substitué ou non substitué, ledit groupe alkyle comprenant un groupe 1-adamantyle et un groupe 2-adamantyle, un groupe alkoxy substitué ou non substitué, ledit groupe alkoxyle comprenant un groupe phénoxy, un groupe 4-tertiobutylphénoxy, un groupe benzyloxy et un groupe thiényloxy, un groupe aryle substitué ou non substitué ou un groupe hétérocyclique substitué ou non substitué, et peuvent être identiques ou différents.

**4.** Amine suivant la revendication 1, dans laquelle X dans la formule générale (1) répond à la formule générale (5) suivante :

X= 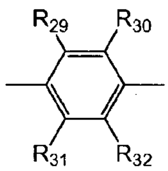 (5)

dans laquelle $R_{29}$ à $R_{32}$ représentent chacun un atome d'hydrogène, un atome d'halogène, un groupe alkyle substitué ou non substitué, ledit groupe alkyle comprenant un groupe 1-adamantyle et un groupe 2-adamantyle, un groupe alkoxy substitué ou non substitué, ledit groupe alkoxyle comprenant un groupe phénoxy, un groupe 4-tertiobutylphénoxy, un groupe benzyloxy et un groupe thiényloxy, un groupe aryle substitué ou non substitué ou un groupe hétérocyclique substitué ou non substitué, et peuvent être identiques ou différents.

**5.** Amine suivant la revendication 1, dans laquelle X dans la formule générale (1) représente une liaison simple.

**6.** Amine suivant la revendication 1, dans laquelle la formule générale (1) représente une formule choisie dans le groupe consistant en les formules chimiques suivantes :

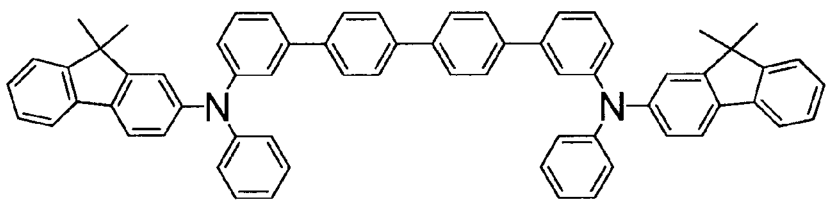

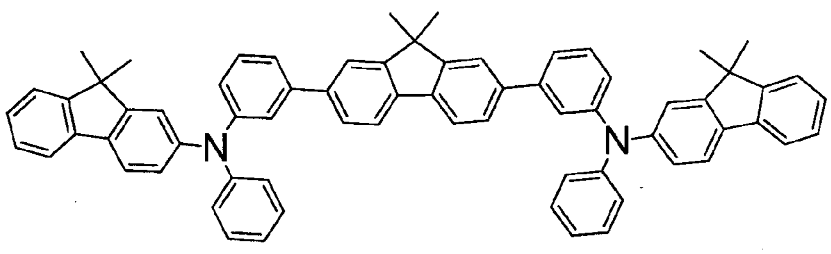

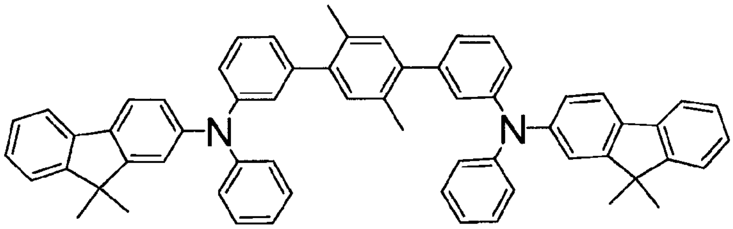

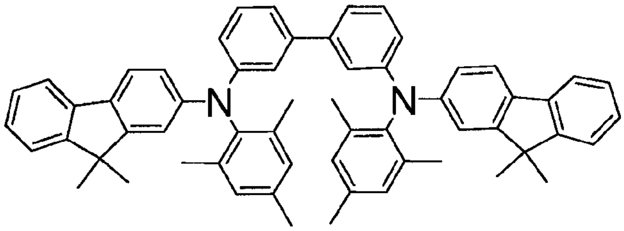

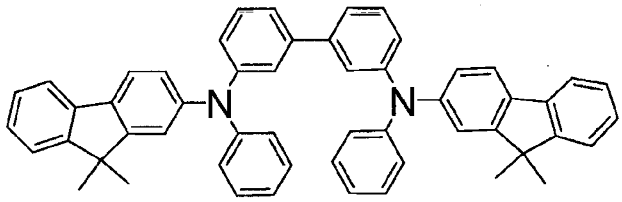
.

7. Dispositif électroluminescent organique, comprenant une couche de composé organique interposée entre deux électrodes constituées d'une anode et d'une cathode, au moins une de l'anode et de la cathode étant transparente ou semi-transparente, dans lequel la couche de composé organique contient une amine suivant la revendication 1.

8. Dispositif électroluminescent organique, comprenant une couche de composé organique comme couche de transport de lacunes ou couche d'injection de lacunes interposée entre deux électrodes constituées d'une anode et d'une cathode, au moins une de l'anode et de la cathode étant transparente ou semi-transparente, dans lequel la couche de transport de lacunes ou la couche d'injection de lacunes contient une amine suivant la revendication 1.

9. Dispositif électroluminescent organique, comprenant une couche de composé organique comme couche électroluminescente interposée entre deux électrodes constituées d'une anode et d'une cathode, au moins une de l'anode et de la cathode étant transparente ou semi-transparente, dans lequel la couche électroluminescente contient une amine suivant la revendication 1.

10. Dispositif comprenant un substrat opaque et le dispositif électroluminescent organique suivant la revendication 7.

11. Dispositif suivant la revendication 10, comprenant en outre un film servant de filtre coloré.

FIG. 1

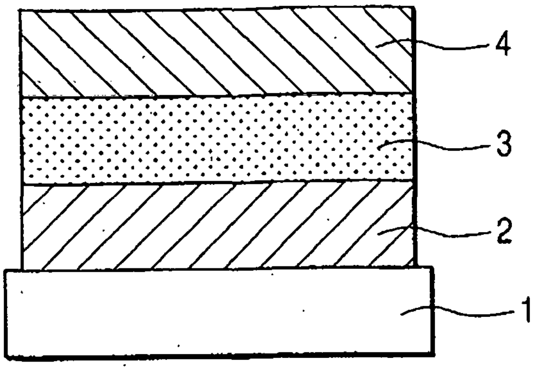


FIG. 2

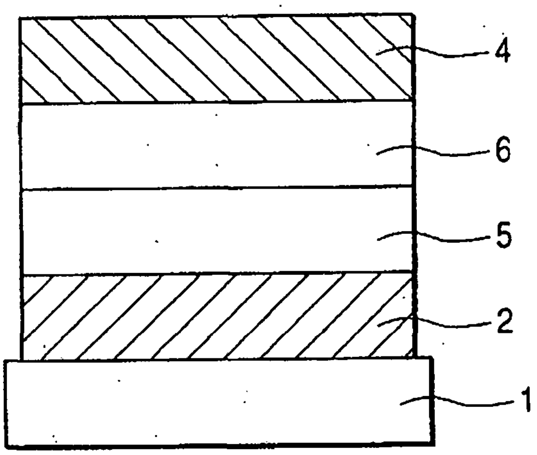


FIG. 3

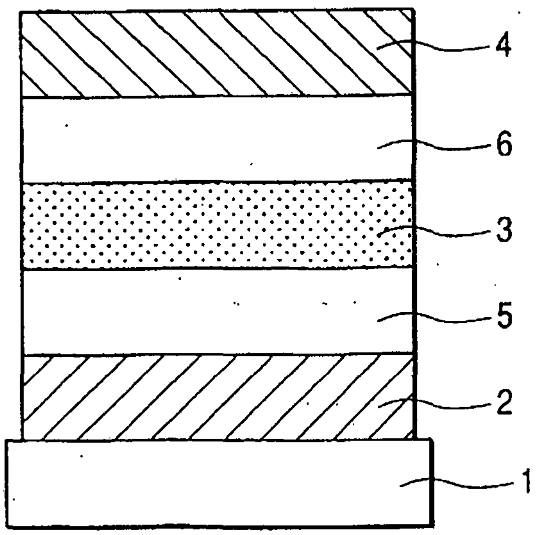

FIG. 4

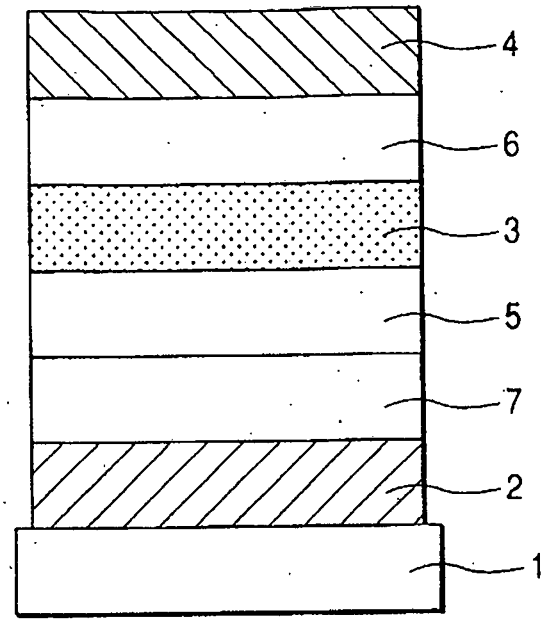


FIG. 5

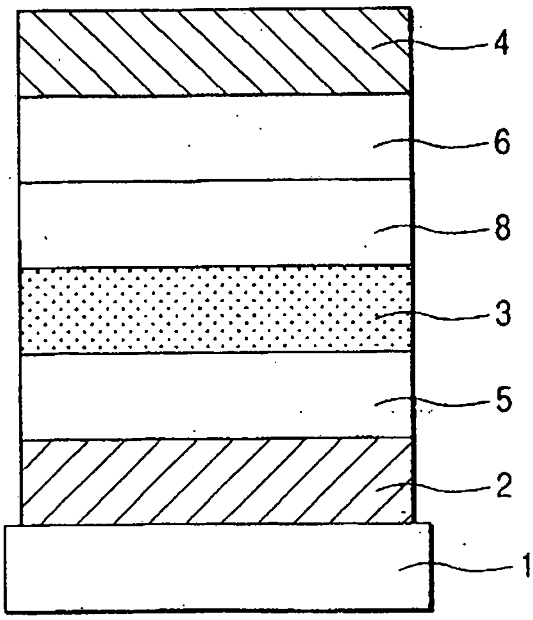

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2851185 B
- JP 3508984 B
- JP 2000016973 A
- JP H10251633 B
- JP 2001039933 A
- JP 2005320277 A
- EP 1577364 A1
- WO 2004020372 A1


### Non-patent literature cited in the description


- *Chem. Rev.,* 1995, vol. 95, 2457-2483
- *Bull. Chem. Soc. Jpn.,* 1978, vol. 51, 2091